# FASCICULE DE BREVET EUROPEEN

(11) **EP 2 155 662 B1**
(45) Date de publication et mention de la délivrance du brevet: **01.12.2010**
(21) Numéro de dépôt: 08805936.5
(22) Date de dépôt: 04.06.2008
(51) Int. Cl.: C07C 233/54, C07C 233/55, C07C 309/65, C07C 309/66, C07C 309/71, C07C 311/32, C07C 311/35, C07C 59/64, C07C 59/68, C07C 69/734, A61P 17/00, A61Q 19/08, A61Q 5/00, A61Q 19/00

(54) **NOUVEAUX DERIVES D'ACIDE 3-PHENYL PROPANOIQUE ACTIVATEURS DES RECEPTEURS DE TYPE PPAR, LEUR METHODE DE PREPARATION ET LEUR UTILISATION DANS DES COMPOSITIONS COSMETIQUES OU PHARMACEUTIQUES**
NEUARTIGE DERIVATE AUS 3-PHENYL-PROPANSÄURE AKTIVIERENDEN PPAR-REZEPTOREN, VERFAHREN ZU IHRER HERSTELLUNG UND IHRE VERWENDUNG IN KOSMETISCHEN ODER PHARMAZEUTISCHEN ZUSAMMENSETZUNGEN
NOVEL DERIVATIVES OF 3-PHENYL PROPANOIC ACID ACTIVATING PPAR-TYPE RECEPTORS, METHOD FOR PREPARING SAME AND USE THEREOF IN COSMETIC OR PHARMACEUTICAL COMPOSITIONS

(30) Priorité: 05.06.2007 FR 0755476
(43) Date de publication de la demande: 24.02.2010
(73) Titulaire: Galderma Research & Development, 06410 Biot (FR)
(72) Inventeur: BOITEAU, Jean-Guy, 34130 Saint-Aunes (FR); CLARY, Laurence, 06480 La Colle Sur Loup (FR); PASCAL, Jean-Claude, 06100 Nice (FR); PARNET, Véronique, 06110 Le Cannet (FR)
(74) Mandataire: Allab, Myriam
(86) Numéro de dépôt international: PCT/FR2008/050996
(87) Numéro de publication internationale: WO 2008/152333

(56) Documents cités:
- WO-A-01/40172
- WO-A-02/12210
- WO-A-99/62871
- WO-A-2007/049158

## Description

L'invention concerne, à titre de produits industriels nouveaux et utiles, une nouvelle classe de dérivés d'acide 3-phényl propanoïque activateurs des récepteurs de type Peroxisome Proliferator-Activated Receptor de sous-type γ (PPARγ). Elle concerne également leur méthode de préparation et leur utilisation dans des compositions pharmaceutiques destinées à un usage en médecine humaine ou vétérinaire, ou bien encore dans des compositions cosmétiques.

L'activité des récepteurs de type PPARs a fait l'objet de nombreuses études. On peut citer à titre indicatif la publication intitulée "Differential Expression of Peroxisome Proliferator-Activated Receptor Subtypes During the Differentiation of Human Keratinocytes", Michel Rivier et al., J. Invest. Dermatol 111, 1998, p 1116-1121, dans laquelle est répertorié un grand nombre de références bibliographiques concernant les récepteurs de type PPARs. On peut également citer à titre indicatif, le dossier intitulé "The PPARs: From orphan receptors to Drug Discovery", Timothy M. Willson, Peter J. Brown, Daniel D. Sternbach, et Brad R. Henke, J. Med.Chem., 2000, Vol.43, p. 527-550.
Les récepteurs PPARs activent la transcription en se liant à des éléments de séquences d'ADN, appelés les éléments de réponse des proliférateurs de peroxysome (PPRE), sous forme d'un hétérodimère avec les récepteurs X des rétinoïdes (appelés les RXRs).

Trois sous-types de PPARs humains ont été identifiés et décrits : les PPARα, PPARγ et PPARδ (ou NUC1).

PPARα est principalement exprimé dans le foie alors que PPARδ est ubiquitaire.
PPARγ est le plus étudié des trois sous-types. L'ensemble des références suggère un rôle critique des PPARγ dans la régulation de la différentiation des adipocytes, où il est fortement exprimé. Il joue également un rôle clé dans l'homéostasie lipidique systémique.

Il a été notamment décrit dans la demande internationale WO 96/33724 que des composés sélectifs des PPARγ, tels qu'une prostaglandine-J2 ou -D2, sont des actifs potentiels pour le traitement de l'obésité et du diabète.

Par ailleurs, la Demanderesse a déjà décrit dans les demandes internationales WO 02/12210, WO 03/055867 et WO 2007/049158 l'utilisation de composés bi-aromatiques activateurs des récepteurs de type PPARγ dans la préparation d'une composition pharmaceutique, la composition étant destinée à traiter les désordres cutanés liés à une anomalie de la différentiation des cellules épidermiques.

Il n'en demeure pas moins qu'il reste nécessaire de rechercher de nouveaux composés présentant une bonne activité et des propriétés pharmaceutiques avantageuses.

La Demanderesse a maintenant identifié de nouveaux dérivés d'acide 3-phényle propanoïque présentant, de façon surprenante, une activité vis-à-vis des récepteurs PPARs gamma.
Les molécules décrites dans le brevet WO 2007/049158 absorbent en UV à des longueurs d'ondes supérieures à 290nm du fait de leurs structures conjuguées. Par contre, pour les composés de la présente invention, il n'y a pas d'absorption dans cette gamme de longueur d'onde (290-700nm). Cette absence d'absorption réduit avantageusement les risques de photo-toxicité et de photo-génotoxicité des composés de la présente invention, ce qui accroit la sécurité pour leur utilisation dans des compositions pharmaceutiques ou cosmétiques appliquées par voie topique.
Par ailleurs, les composés selon la présente invention sont obtenus, le plus souvent, sous forme solide, ce qui présente l'avantage de pouvoir opérer facilement à leur purification à l'échelle industrielle en utilisant des techniques comme la recristallisation. L'utilisation de composés solides pour la préparation de compositions pharmaceutiques et/ou cosmétiques présente également un réel avantage dans le cadre de leur développement pharmaceutique et/ou cosmétique en raison du taux de solvants résiduels quasi nul que ces composés contiennent comparativement à celui qu'ils peuvent contenir lorsqu'ils se présentent sous forme d'huile.

Ainsi, la présente invention concerne des composés répondant à la formule générale (I) suivante : 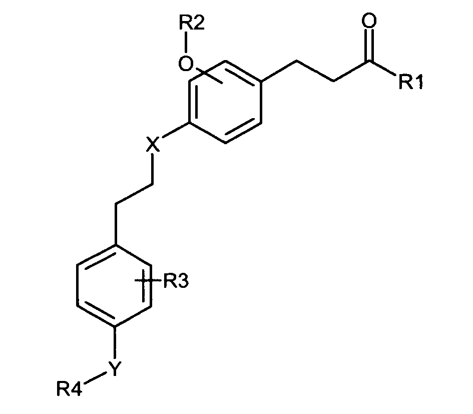 dans laquelle :
- R₁ représente un radical hydroxyle ou un radical alkoxy ;
- R₂ représente un hydrogène, un radical alkyle, un radical cycloalkyle, un radical aralkyle éventuellement substitué ou un radical polyether ;
- R₃ représente un hydrogène, un halogène, un radical alkyle ou un radical alkoxy ;
- R₄ représente un radical alkyle, un radical aryle éventuellement substitué ou un radical aralkyle éventuellement substitué ;
- X représente un atome d'oxygène ou un radical CH₂,
- Y représente un atome d'oxygène, un radical NR₅, un radical OSO₂, OCO, NR₅CO ou NR₅SO₂ ;
- R₅ représente un atome d'hydrogène ou un radical alkyle ;
ainsi que ses sels avec un acide ou une base pharmaceutiquement acceptable, ses solvates pharmaceutiquement acceptables et ses hydrates.

En particulier, lorsque les composés selon l'invention se présentent sous forme d'un sel, il s'agit d'un sel d'un métal alcalin, en particulier, un sel de sodium ou de potassium, ou d'un sel d'un metal alcalino-terreux, en particulier le magnésium ou le calcium, ou encore d'un sel avec une amine organique, plus particulièrement, avec un acide aminé tel que l'arginine ou la lysine.

Lorsque les composés selon l'invention possèdent une fonction amine et se présentent sous la forme d'un sel de cette amine, il s'agit d'un sel d'acide inorganique comme par exemple l'acide chlorhydrique, l'acide sulfurique, ou l'acide bromhydrique ou d'un sel d'acide organique comme par exemple l'acide acétique, l'acide triflique, l'acide tartrique, l'acide oxalique, l'acide citrique, l'acide trifuoroacétique ou l'acide méthane sulfonique.

Selon la présente invention, par radical alkyle, on entend une chaîne hydrocarbonée saturée, linéaire ou ramifiée, comprenant de 1 à 12 atomes de carbone et, plus particulièrement, de 1 à 6 atomes de carbone.
De préférence, les radicaux alkyles mis en oeuvre dans le cadre de la présente invention sont choisis parmi les radicaux méthyle, éthyle, propyle, isopropyle, butyle, isobutyle, tertiobutyle, pentyle, isoamyle, amyle, hexyle, heptyle, octyle et décyle. Plus particulièrement, les radicaux alkyles sont choisis parmi les radicaux méthyle, éthyle, propyle, isopropyle, butyle, isobutyle, tertiobutyle, pentyle, isoamyle, amyle et hexyle.

Selon la présente invention, par radical alkyle inférieur, on entend un radical alkyle tel que précédemment défini et comprenant de 1 à 4 atomes de carbone et, avantageusement, 1 à 3 atomes de carbone. Ainsi, de préférence, de tels radicaux sont choisis parmi les radicaux méthyle, éthyle, propyle, isopropyle, butyle, isobutyle et tertiobutyle.

Selon, la présente invention, par radical cycloalkyle, on entend une chaîne hydrocarbonée saturée, cyclique, comprenant de 3 à 7 atomes de carbone.
De préférence, le radical cycloalkyle est choisi parmi les radicaux cyclopropyle, cyclobutyle, cyclopentyle, cyclohexyle et cycloheptyle.

Selon la présente invention, par radical aryle, on entend un phenyle ou un naphtyle non substitué.

Selon la présente invention, par radical aryle substitué, on entend un phényle ou un naphtyle substitué par un ou plusieurs atomes ou groupes d'atome choisis parmi : alkyle, alkoxy, halogène, hydroxy, cyano, trifluorométhyle et nitro.
De préférence, le radical aryle substitué est choisi parmi les radicaux phényles monosubstitués par un halogène.

Selon la présente invention, par radical aralkyle, on entend un alkyle substitué par un phényle ou un napthyle non substitué.
De préférence, le radical aralkyle est un radical benzyle ou phénéthyle.

Selon la présente invention, par radical aralkyle substitué, on entend un radical aralkyle substitué par un ou plusieurs atomes ou groupes d'atome choisis parmi : alkyle, alkoxy, halogène, hydroxy, cyano, trifluorométhyle et nitro.

Le radical aralkyle substitué est de préférence choisi parmi les radicaux phénéthyles monosubstitués par un radical alkyle inférieur et les radicaux benzyles monosubstitués par un halogène.

Selon la présente invention, par atome d'halogène, on entend un atome de fluor, de chlore, de brome ou d'iode.

Selon la présente invention, par radical hydroxyle, on entend le radical -OH.

Selon la présente invention, par radical alkoxy, on entend un atome d'oxygène substitué par un alkyle.
Les radicaux alkoxy sont de préférence les radicaux méthoxy, éthoxy, isopropyloxy, n-propyloxy, tertio-butoxy, n-butoxy, n-pentyloxy et n-hexyloxy.

Selon la présente invention, par radical alkoxy inférieur, on entend un atome d'oxygène substitué par un alkyle inférieur.

Selon la présente invention, par radical polyéther, on entend un radical ayant de 1 à 7 atomes de carbone interrompu par au moins un atome d'oxygène. De préférence, le radical polyéther est choisi parmi les radicaux tels que méthoxyéthoxy, éthoxyéthoxy, méthoxyéthyle, éthoxyéthyle ou méthoxyéthoxyéthoxy.

Parmi les composés de formule générale (I) ci-dessus rentrant dans le cadre de la présente invention, on peut notamment citer les composés suivants (seuls ou en mélange) :
1. acide 3-{4-[3-(4-benzyloxy-3-methoxy-phenyl)-propyl]-3-butoxy-phenyl}-propanoique
2. acide 3-{3-butoxy-4-[3-(4-ethoxy-3-methoxy-phenyl)-propyl]-phenyl}-propanoique
3. acide 3-{3-butoxy-4-[3-(4-butoxy-3-methoxy-phenyl)-propyl]-phenyl}-propanoique
4. acide 3-{3-butoxy-4-[3-(4-methanesulfonyloxy-3-methoxy-phenyl)-propyl]-phenyl}-propanoique
5. acide 3-(4-{3-[4-(butane-1-sulfonyloxy)-3-methoxy-phenyl]-propyl}-3-butoxy-phenyl)-propanoique
6. acide 3-{3-butoxy-4-[3-(4-ethanesulfonyloxy-3-methoxy-phenyl)-propyl]-phenyl)-propanoique
7. acide 3-[4-{3-[4-(butane-1-sulfonyloxy)-3-methoxy-phenyl]-propyl}-3-(3-fluoro-benzyloxy)-phenyl]-propanoique
8. acide 3-[4-{3-[4-(butane-1-sulfonyloxy)-3-methoxy-phenyl]-propyl} -3-(4-fluoro-benzyloxy)-phenyl]-propanoique
9. acide 3-(4-{3-[4-(butane-1-sulfonyloxy)-3-methoxy-phenyl]-propyl}- 3-cyclopropylmethoxyphenyl)-propanoique
10. acide 3-[4-{3-[4-(butane-1-sulfonyloxy)-3-methoxy-phenyl]-propyl}-2-(3-methoxybenzyloxy)-phenyl]-propanoique
11. acide 3-(4-{3-[4-(butane-1-sulfonyloxy)-3-methoxy-phenyl]-propyl}-2-butoxy-phenyl)-propanoique
12. 3-[4-[3-(4-acétylamino-phenyl)-propyl]-3-(2-methoxy-ethoxy)-phenyl]-propanoate de méthyle
13. 3-(4-{3-[4-(acétyl-methyl-amino)-phenyl]-propyl}-3-methoxy-phenyl)-propanoate de méthyle
14. acide 3-(4-{3-[4-(butane-1-sulfonyloxy)-phenyl]-propyl}-3-hydroxy-phenyl)-propanoique
15. acide 3-(4-{3-[4-(butane-1-sulfonylamino)-phenyl]-propyl}-3-butoxy-phenyl)-propanoique
16. acide 3-[4-(2-{4-[(3-chloro-benzoyl)-methyl-amino]-phenyl}-ethoxy)-3-(2-ethoxy-ethoxy)-phenyl]-propanoique
17. acide 3-[3-butoxy-4-(2-{4-[methyl-(2-p-tolyl-ethanesulfonyl)-amino]-phenyl}-ethoxy)-phenyl]-propanoique
18. acide 3-(4-{3-[4-(butane-1-sulfonyloxy)-3-methoxy-phenyl]-propyl}-3-butoxy-phenyl)-propanoique
19. 3-{3-butoxy-4-[3-(4-ethoxy-3-fluoro-phenyl)-propyl]-phenyl}-propanoate de méthyle
20. acide 3-[4-{3-[4-(butane-1-sulfonyloxy)-2-methoxy-phenyl]-propyl}-3-(2-ethoxy-ethoxy)-phenyl]-propanoique
21. acide 3-(4-{3-[3-chloro-4-(hexane-1-sulfonyloxy)-phenyl]-propyl}-3-ethoxy-phenyl)-propanoique
22. acide 3-{4-[2-(3-chloro-4-ethoxy-phenyl)-ethoxy]-3-methoxy-phenyl}-propanoique
23. butyrate de 4-{3-[4-(2-carboxy-ethyl)-2-methoxy-phenyl]-propyl}-phenyle

Selon la présente invention, les composés préférés répondant à la formule générale (I) sont ceux qui présentent l'une au moins des caractéristiques suivantes :
- R₁ est un radical hydroxyle,
- R₂ représente un radical alkyle ou un radical polyéther,
- R₃ représente un atome d'hydrogène, un radical alkoxy ou un halogène,
- R₄ représente un radical alkyle,
- X représente un atome d'oxygène ou un groupement CH₂,
- Y représente un enchaînement -NR₅SO₂ ou un enchaînement -OSO₂, R₅ étant tel que précédemment défini.

Selon la présente invention, les composés tout particulièrement préférés répondant à la formule générale (I) sont ceux qui présentent l'une au moins des caractéristiques suivantes :
- R₁ est un radical hydroxyle,
- R₂ représente un radical alkyle inférieur,
- R₃ représente un radical alkoxy inférieur,
- R₄ représente un radical alkyle inférieur,
- X représente un atome d'oxygène ou un groupement CH₂,
- Y représente un enchaînement -OSO₂.

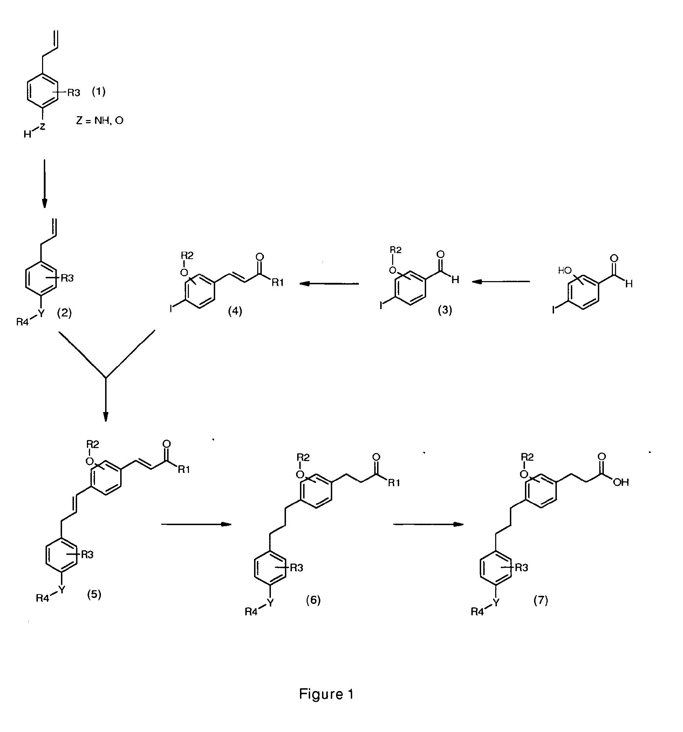

Une description générale de méthodes de préparation des composés de formule générale (I) est donnée ci-après, en référence aux schémas des figures 1, 2, 3 et 4. Sur ces schémas et dans la description du procédé qui va suivre, à moins qu'il ne soit spécifié autrement, tous les substituants sont tels que définis pour les composés de formule (I).

Comme présenté sur la figure 1, les composés de formule générale (I) pour lesquels X= CH₂ peuvent être obtenus à partir des intermédiaires de formule générale (5): 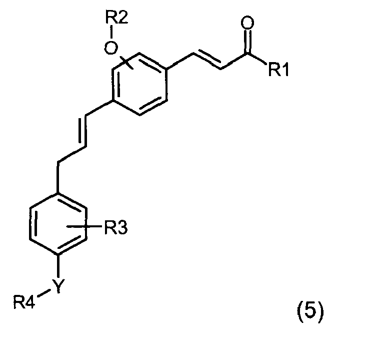 L'obtention des intermédiaires de formule générale (5) peut être réalisée par réaction de Heck entre les composés de formule générale (2) 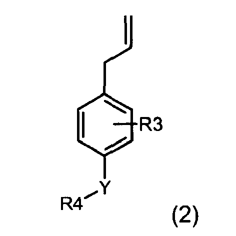 et les composés iodés de formule générale (4) 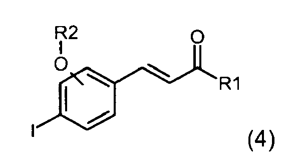 en présence d'un catalyseur au palladium, par exemple du palladium (II) acétate, et d'une phosphine. Les composés de formule générale (2) sont obtenus à partir des composés de formule générale (1) 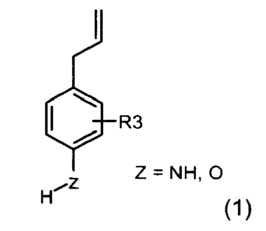 selon les étapes suivantes :
a) soit une addition sur un chlorure d'acide sulfonique (R₄SO₂Cl)
b) soit une addition sur un halogénure d'acide carboxylique (par exemple R₄COCl)
c) soit une réaction avec un dérivé halogéné (par exemple R₄Br ou R₄Cl) en présence d'une base telle que l'hydrure de sodium ou le carbonate de potassium.

Les dérivés ainsi obtenus peuvent éventuellement être alkylés par réaction avec un dérivé halogéné (par exemple R₅Br ou R₅Cl) en présence d'une base telle que l'hydrure de sodium ou le carbonate de potassium.

Le procédé conduisant aux composés de formule générale (4) à partir du 3-hydroxy-4-iodobenzaldéhyde ou du 2-hydroxy-4-iodobenzaldéhyde commercial comprend les deux étapes suivantes :
a) alkylation du 3-hydroxy-4-iodobenzaldéhyde ou du 2-hydroxy-4-iodobenzaldéhyde en présence d'une base (carbonate de potassium par exemple) et d'un dérivé halogéné (par exemple R₂Br ou R₂Cl) pour conduire aux dérivés aldéhyde (3). 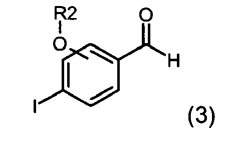
b) une réaction de Wittig ou de Horner-Emmons entre leurs précurseurs aldéhydes (3) et les phosphonates (par exemple (diéthoxy-phosphoryl)-acétate d'éthyle) ou phosphoniums (par exemple, chlorure de (triphényl-phosphonium)-acétate de méthyle) correspondants pour conduire aux composés de formule générale (4).

Après réduction des doubles liaisons des composés de formule générale (5), les composés (6) sont obtenus, 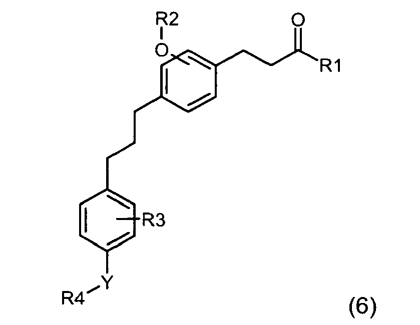 puis saponifiés en présence d'hydroxyde de sodium par exemple dans un mélange de tétrahydrofurane et d'eau ou d'acétone et d'eau par exemple pour conduire aux composés de formule générale (7).

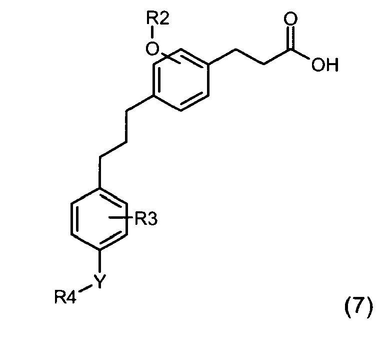

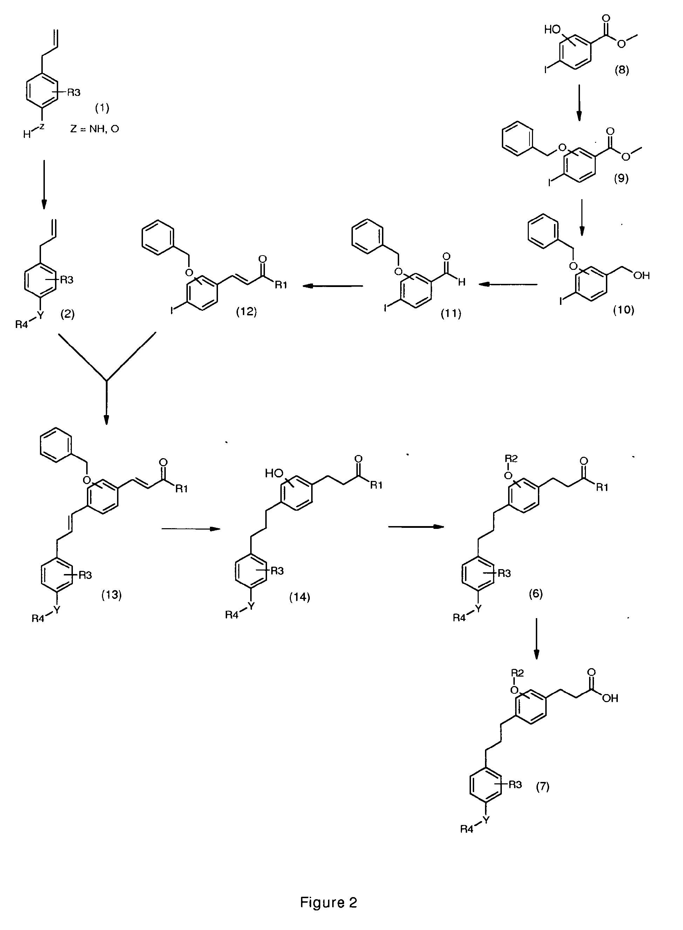

Le schéma de la figure 2 décrit une autre méthode d'obtention des composés de formule générale (6).

Par une réaction de Heck entre les dérivés (2) et les dérivés iodés (12) (correspondant aux composés (4) de la figure 1 pour lesquels R₂ est un benzyle), 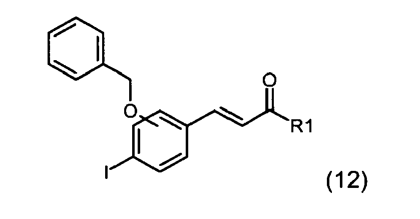 en présence d'un catalyseur au palladium par exemple du palladium (II) acétate et d'une phosphine, les composés de formule générale (13) sont obtenus.

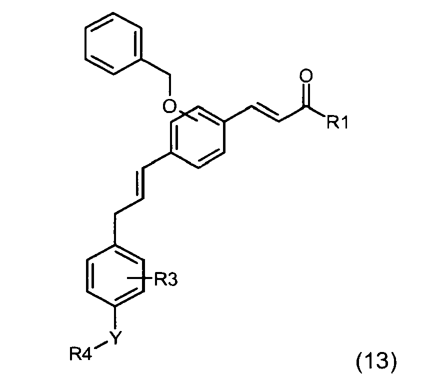

Après réduction des doubles liaisons et déprotection du phénol (coupure de l'éther benzylique), on obtient les composés de formule générale (14).

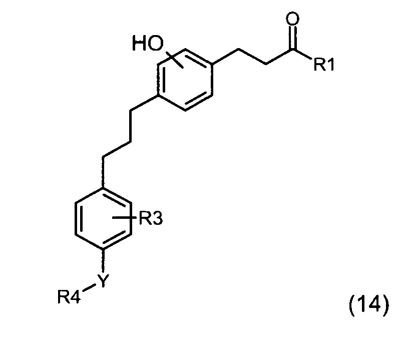

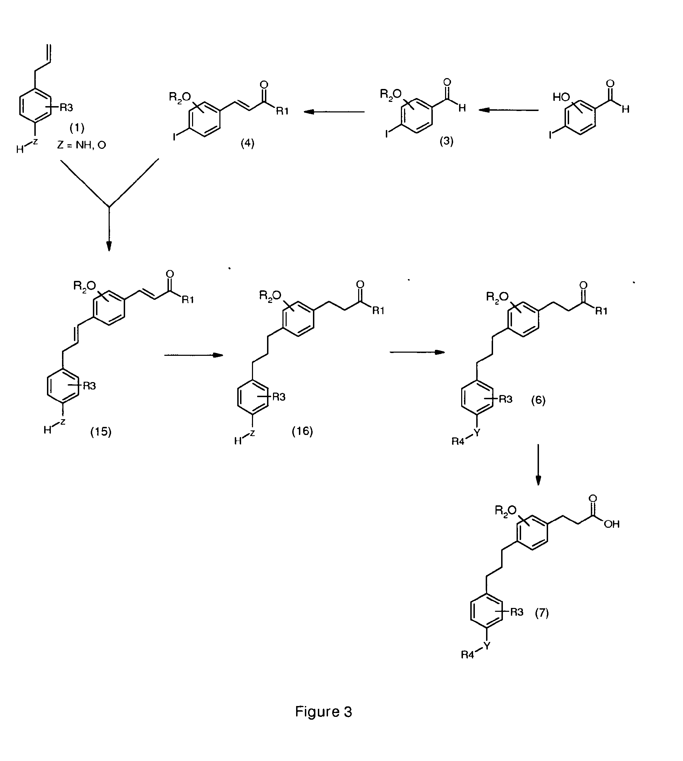

Par réaction d'alkylation des composés de formule générale (14) avec un dérivé halogéné (par exemple R₂Br ou R₂I) en présence d'une base telle que l'hydrure de sodium ou le carbonate de potassium par exemple, les composés de formule générale (6) sont obtenus. 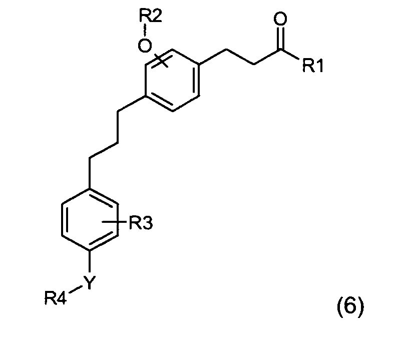 Une troisième méthode d'obtention des composés de formule générale (6) est décrite dans la figure 3 à partir d'une réaction de Heck entre un dérivé de type 4-allyl-phénylamine ou 4-allyl-phénol de formule générale (1) éventuellement substitué par un groupement R₃ 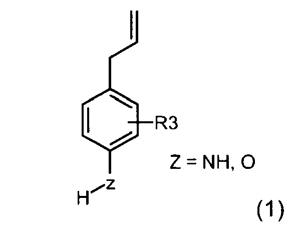 et un dérivé iodé de formule générale (4) pour conduire au composé de formule générale (15). 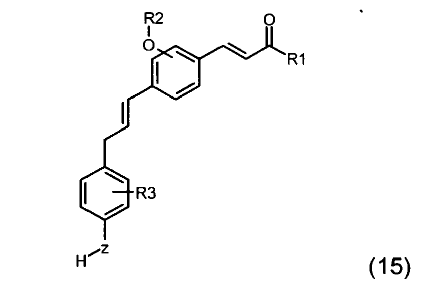 Après réduction des doubles liaisons pour conduire aux composés de formule générale (16) 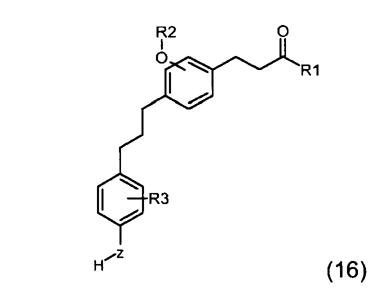 le procédé conduisant aux composés de formule générale (6) comprend les étapes suivantes :
a) soit une addition sur un chlorure d'acide sulfonique (R₄SO₂Cl)
b) soit une addition sur un halogénure d'acide carboxylique (par exemple R₄COCl)
c) soit une réaction avec un dérivé halogéné (par exemple R₄Br ou R₄Cl) en présence d'une base telle que l'hydrure de sodium ou le carbonate de potassium par exemple.

Les dérivés ainsi obtenus peuvent éventuellement être alkylés par réaction avec un dérivé halogéné (par exemple R₅Br ou R₅Cl) en présence d'une base telle que l'hydrure de sodium ou le carbonate de potassium.

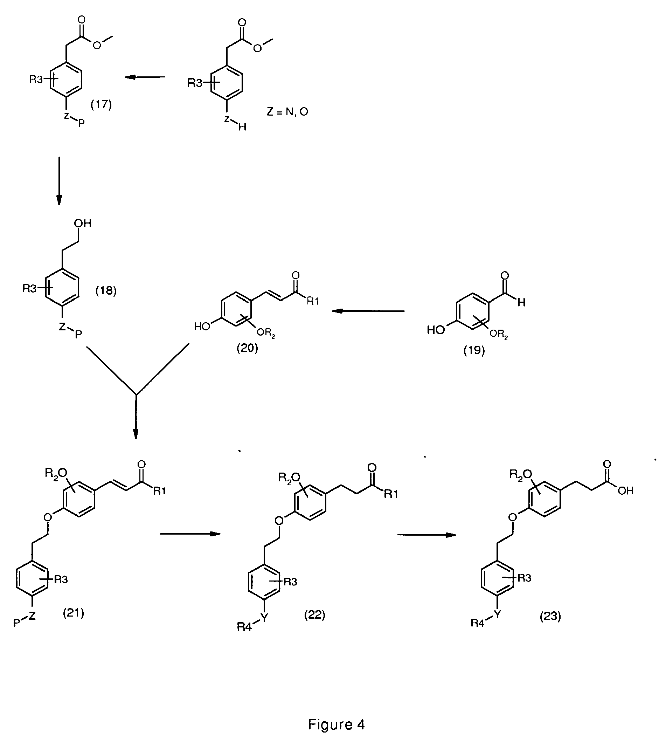

Une description générale de préparation des composés de formule (I) pour lesquels X= O est illustrée dans la figure 4 et détaillée ci-après :

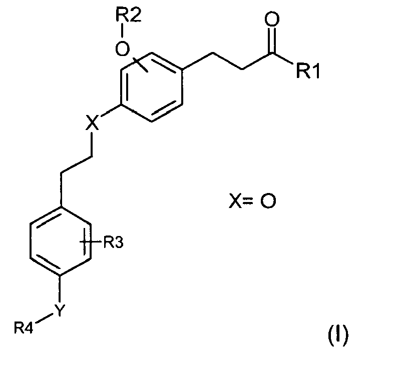

Comme présenté sur la figure 4, les composés de formule générale (I) pour lesquels X= O peuvent être obtenus à partir des intermédiaires de formule générale (21):

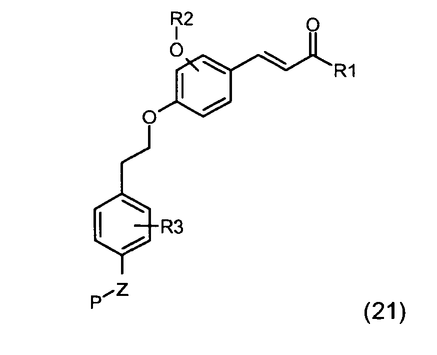

L'obtention des dérivés de formule générale (21) peut être réalisée par réaction de Mitsunobu entre les composés de formule générale (18) 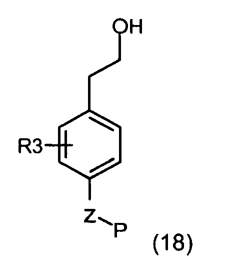 et les composés de formule générale (20) 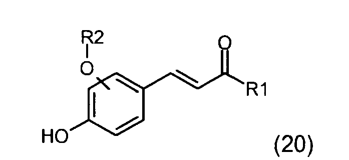 en présence de triphénylphosphine et de diéthylazodicarboxylate par exemple.

Le procédé permettant la synthèse des composés de formule générale (18) à partir de dérivés commerciaux de type (4-amino-phenyl)-acétate de méthyle ou (4-hydroxy-phenyl)-acétate de méthyle éventuellement subsitués par un groupement R₃ comprend les étapes suivantes :
a) protection de la fonction amine ou hydroxyle pour conduire aux composés de formule générale (17) 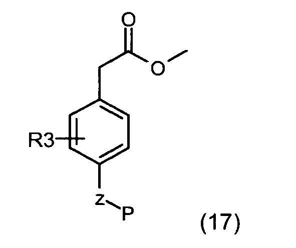
b) réduction de la fonction ester en alcool en présence d'un réducteur tel que le borohydrure de lithium par exemple

Les composés de formule générale (20) peuvent être obtenus par une réaction de Wittig ou de Horner-Emmons entre leurs précurseurs aldéhydes de formule générale (19) (préalablement préparé par réaction de butanolate de sodium sur le 3-bromo-4-hydroxy-benzaldehyde ou 2-bromo-4-hydroxy-benzaldéhyde commercial en présence de chlorure de cuivre (I)) et les phosphonates (par exemple (diéthoxy-phosphoryl)-acétate d'éthyle) ou phosphoniums (par exemple, chlorure de (triphényl-phosphonium)-acétate de méthyle).

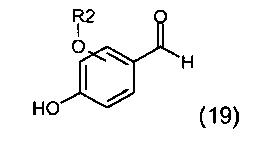

Le procédé conduisant des composés de formule générale (21) aux composés de formule générale (23) comprend les étapes suivantes :
a) déprotection de la fonction alcool ou amine
b) soit une addition sur un chlorure d'acide sulfonique (R₄SO₂Cl) soit une addition sur un halogénure d'acide carboxylique (par exemple R₄COCl) soit une réaction avec un dérivé halogéné (par exemple R₄Br ou R₄Cl) en présence
   d'une base telle que l'hydrure de sodium ou le carbonate de potassium, par exemple. Les dérivés ainsi obtenus peuvent éventuellement être alkylés par réaction avec un dérivé halogéné (par exemple R₅Br ou R₅Cl) en présence d'une base telle que l'hydrure de sodium ou le carbonate de potassium.
c) réduction de la double liaison pour conduire aux composés de formule générale (22),
d) saponification en présence d'hydroxyde de sodium par exemple dans un mélange de tétrahydrofurane et d'eau ou d'acétone et d'eau pour conduire aux composés de formule générale (23).

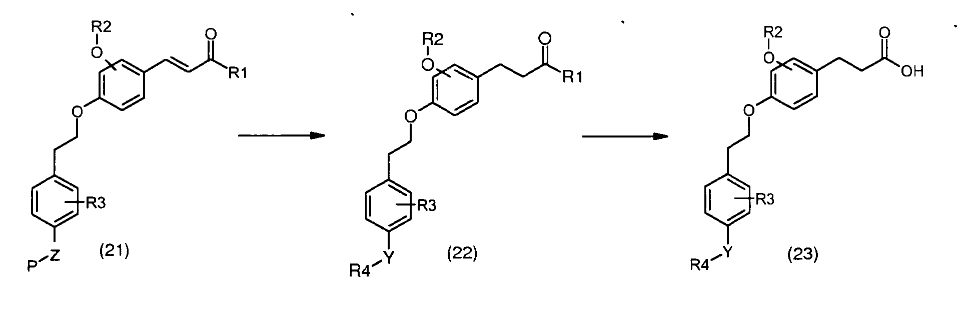

Les groupes fonctionnels éventuellement présents dans les intermédiaires réactionnels utilisés dans le procédé peuvent être protégés, soit sous forme permanente, soit sous forme temporaire, par des groupes protecteurs qui assurent une synthèse univoque des composés attendus. Les réactions de protection et déprotection sont effectuées selon des techniques bien connues de l'homme de l'art. Par groupe protecteur temporaire des amines, alcools ou des acides carboxyliques on entend les groupes protecteurs tels que ceux décrits dans « Protective Groups in Organic Chemistry », ed McOmie J. W. F., Plenum Press, 1973, dans « Protective Groups in Organic Synthesis », 2nde édition, Greene T.W. et Wuts P.G.M., ed John Wiley et Sons, 1991 et dans « Protecting Groups », Kocienski P.J., 1994, Georg Thieme Verlag.

Les composés selon l'invention présentent des propriétés modulatrices des récepteurs de type PPARs. Cette activité sur les récepteurs PPARα, δ et γ est mesurée dans un test de transactivation et quantifiée par la constante de dissociation Kdapp (apparent), tel que décrit à l'exemple 10.
Les composés préférés de la présente invention présentent une constante de dissociation inférieure ou égale à 1000 nM, et avantageusement inférieure ou égale à 200 nM.

De préférence, les composés sont des modulateurs des récepteurs de type PPARγ spécifique, c'est à dire qu'ils présentent un rapport entre le Kdapp pour les récepteurs PPARα ou PPARδ, et le Kdapp pour les récepteurs PPARγ, supérieur ou égal à 10. De préférence, ce rapport PPARα/PPARγ ou PPARδ/PPARγ est supérieur ou égal à 50 et plus avantageusement supérieur ou égal à 100.

La présente invention a également pour objet à titre de médicament les composés de formule générale (I) tels que décrits ci-dessus.

Ainsi les composés tels que décrits précédemment selon l'invention peuvent être utilisés dans la fabrication d'un médicament destiné à réguler et/ou à restaurer le métabolisme des lipides cutanés.
L'invention concerne également un produit choisi parmi les composés de formule (i) pour son utilisation dans le traitement et/ou la prévention des désordres décrits ci-dessous.

Les composés selon l'invention conviennent particulièrement bien dans les domaines de traitement suivants :
1) pour traiter les affections dermatologiques liées à un désordre de la kératinisation portant sur la différenciation et sur la prolifération, notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que toute lésion précancéreuse cutanées telles que les kératoacanthomes,
5) pour traiter d'autres désordres dermatologiques tels que les dermatoses immunes telles le lupus érythémateux, les maladies immunes bulleuses et les maladies du collagène, telle la sclérodermie,
6) dans le traitement d'affections dermatologiques ou générales à composante immunologique,
7) dans le traitement de désordres cutanés dus à une exposition aux rayonnements U.V. ainsi que pour réparer ou lutter contre le vieillissement de la peau, qu'il soit photo-induit ou chronologique, ou pour réduire les pigmentations et les kératoses actiniques, ou toutes pathologies associées au vieillissement chronologique ou actinique, telle la xérose,
8) pour lutter contre les troubles de la fonction sébacée tels que l'hyperséborrhée de l'acné ou la séborrhée simple,
9) pour prévenir ou traiter les troubles de la cicatrisation, ou pour prévenir ou pour réparer les vergetures,
10) dans le traitement des désordres de la pigmentation, tel l'hyperpigmentation, le mélasma, l'hypopigmentation ou le vitiligo,
11) dans le traitement des affections du métabolisme des lipides, tel l'obésité, l'hyperlipidémie, ou le diabète non insulino-dépendant,
12) dans le traitement d'affections inflammatoires telles que l'arthrite,
13) dans le traitement ou la prévention des états cancéreux ou précancéreux,
14) dans la prévention ou le traitement de l'alopécie de différentes origines, notamment l'alopécie due à la chimiothérapie ou aux rayonnements,
15) dans le traitement des troubles du système immunitaire, tel l'asthme, le diabète sucré de type I, la sclérose en plaque, ou autres disfonctionnements sélectifs du système immunitaire,
16) dans le traitement d'affections du système cardiovasculaire telles que l'artériosclérose ou l'hypertension.

La présente invention a également pour objet une composition pharmaceutique ou cosmétique comprenant, dans un milieu physiologiquement acceptable, au moins un composé de formule générale (I) tel que défini ci-dessus.
Par milieu physiologiquement acceptable, on entend un milieu compatible avec la peau, les muqueuses et les phanères.

La présente invention a aussi pour objet l'utilisation des composés de formule générale (I) pour fabriquer un médicament destiné au traitement des affections susmentionnées, en particulier pour réguler et/ou restaurer le métabolisme des lipides cutanés.

L'administration de la composition selon l'invention peut être effectuée par voie orale, enterale, parentérale topique ou oculaire. De préférence, la composition pharmaceutique est conditionnée sous une forme convenant à une application par voie topique. Par voie topique, on entend une administration sur la peau et/ou les phanères.

Par voie orale, la composition, plus particulièrement la composition pharmaceutique, peut se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, la composition peut se présenter sous forme de solutions ou suspensions pour perfusion ou pour injection.

Les composés selon l'invention sont généralement administrés à une dose journalière d'environ 0,001 mg/kg à 100 mg/kg de poids corporel, en 1 à 3 prises.

Les composés sont utilisés par voie systémique à une concentration généralement comprise entre 0,001 et 10% en poids, de préférence entre 0,01 et 1% en poids, par rapport au poids de la composition.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de microsphères ou nanosphères ou vésicules lipidiques ou polymériques ou de patches polymériques et d'hydrogels permettant une libération contrôlée. Cette composition par voie topique peut se présenter sous forme anhydre, sous forme aqueuse ou sous la forme d'une émulsion.

Les composés sont utilisés par voie topique à une concentration généralement comprise entre 0,001 et 10% en poids, de préférence entre 0,01 et 1% en poids, par rapport au poids total de la composition.

Les composés de formule générale (I) selon l'invention trouvent également une application dans le domaine cosmétique, en particulier dans l'hygiène corporelle et capillaire et plus particulièrement pour réguler et/ou restaurer le métabolisme des lipides cutanés.

L'invention a donc également pour objet l'utilisation cosmétique d'un composé de formule (i) ou d'une composition comprenant, dans un milieu physiologiquement acceptable, au moins un des composés de formule générale (I) pour l'hygiène corporelle ou capillaire.

La composition cosmétique selon l'invention contenant, dans un milieu cosmétiquement acceptable, au moins un composé de formule générale (I) ou l'un de ses isomères optiques ou géométriques ou l'un de ses sels, peut se présenter notamment sous forme d'une crème, d'un lait, d'une lotion, d'un gel, de microsphères ou nanosphères ou vésicules lipidiques ou polymériques, d'un savon ou d'un shampooing.

La concentration en composé de formule générale (I) dans la composition cosmétique est comprise entre 0,001 et 3 % en poids, par rapport au poids total de la composition.

Les compositions telles que décrites précédemment peuvent bien entendu en outre contenir des additifs inertes ou même pharmacodynamiquement actifs ou des combinaisons de ces additifs, et notamment, des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétoconazole ou les polyméthylène-4,5 isothiazolidones-3; des antibactériens, des caroténoïdes et, notamment, le β-carotène; des agents anti-psoriatiques tels que l'anthraline et ses dérivés; les acides eicosa-5,8,11,14-tétraynoïque et eicosa-5,8,11-triynoïque, leurs esters et amides et enfin les rétinoïdes. Les composés de formule générale (I) peuvent également être combinés avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques.

Ces compositions peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

A titre d'illustration et sans aucun caractère limitatif, sont donnés ci-après plusieurs exemples d'obtention de composés actifs de formule générale (I) selon l'invention, ainsi que des résultats d'activité biologique de tels composés et diverses formulations concrètes à base de ces composés.

### Exemple 1 : acide 3-{4-[3-(4-benzyloxy-3-methoxy-phenyl)-propyl]-3-butoxy-phenyl}-propanoique

### a- 3-butoxy-4-iodo-benzaldéhyde

21,5ml (189 mmoles) de 1-iodobutane sont ajoutés sur une solution de 31g (126 mmoles) de 3-hydroxy-4-iodo-benzaldéhyde dans 350ml de méthyléthylcétone en présence de 52,2g (378 mmoles) de carbonate de potassium. Le milieu réactionnel est chauffé à 85°C pendant 2 heures. Le solide est filtré et le solvant est évaporé. Le solide obtenu est lavé à l'heptane et 38g (99%) de 3-butoxy-4-iodo-benzaldéhyde sous forme de cristaux blancs sont obtenus.

### b- (E)-3-(3-butoxy-4-iodo-phenyl)-acrylate de méthyle

65,1g (195 mmoles) de (triphénylphosphoranylidène) acétate de méthyle sont ajoutés sur une solution de 29,6g (97 mmoles) de 3-butoxy-4-iodo-benzaldéhyde dans 360ml de toluène. Le mélange réactionnel est chauffé à reflux pendant 2 heures. Le solvant est évaporé et l'huile obtenue est purifié par chromatographie sur colonne de silice élué avec un mélange heptane/dichlorométhane 50/50. 30,5g (87%) de (E)-3-(3-butoxy-4-iodo-phényl)-acrylate de méthyle sont obtenus sous forme de cristaux jaune pâle.

### c- (E)-3-{3-butoxy-4-[(E)-3-(4-hydroxy-3-methoxy-phenyl)-propenyl]-phenyl}-acrylate de méthyle

Une solution de 1,1g (12,1 mmole) d'eugénol, 2,0g (5,5 mmoles) de (E)-3-(3-butoxy-4-iodo-phenyl)-acrylate de méthyle, 24mg (0,1 mmole) de palladium (II) acétate, 77mg (0,2 mmole) de 2-(dicyclohexylphosphino)biphényle dans 15ml d'un mélange diméthylformamide/ triéthylamine 6/1 est agité à 90°C pendant 3 heures. Après addition d'eau, le milieu réactionnel est extrait avec de l'acétate d'éthyle. Les phases organiques sont lavées avec une solution aqueuse saturée de chlorure de sodium, séchées sur sulfate de sodium, filtrées, évaporées. L'huile résiduelle est purifiée par chromatographie sur colonne de silice éluée avec un mélange heptane / acétate d'éthyle 8/2.

### d- 3-{3-butoxy-4-[3-(4-hydroxy-3-methoxy-phenyl)-propyl]-phenyl}-propanoate de méthyle

500mg de Pd/C sont ajoutés sur une solution de (E)-3-{3-butoxy-4-[(E)-3-(4-hydroxy-3-méthoxy-phenyl)-propenyl]-phenyl}-acrylate de méthyle dans 25ml de méthanol. Le milieu réactionnel est agité sous une atmosphère d'hydrogène, à température ambiante pendant 3 heures. Après filtration du catalyseur et évaporation du solvant, 2,0g (90%) de 3-{3-butoxy-4-[3-(4-hydroxy-3-methoxy-phenyl)-propyl]-phenyl}-propanoate de méthyle sont obtenus sous forme d'une huile incolore.

### e- 3-{4-[3-(4-benzyloxy-3-methoxy-phenyl)-propyl]-3-butoxy-phenyl}-propanoate de méthyle

0,2g (0,75 mmoles) de carbonate de potassium puis 0,2ml (0,75 mmoles) de bromure de benzyle sont ajoutés sur une solution de 0,25g (0,62 mmoles) de 3-{3-butoxy-4-[3-(4-hydroxy-3-methoxy-phenyl)-propyl] -phenyl}-propanoate de méthyle dans 25ml de méthyléthylcétone. Le mélange réactionnel est chauffé à 70°C pendant 24 heures puis refroidi, dilué avec de l'acétate d'éthyle, lavé avec une solution aqueuse saturée de chlorure de sodium. La phase organique est séchée sur sulfate de sodium, filtrée, évaporée. Le résidu obtenu est purifié par chromatographie sur colonne de silice élué avec un mélange heptane/acétate d'éthyle 80/20. 276mg (93%) de 3-{4-[3-(4-benzyloxy-3-methoxy-phenyl)-propyl]-3-butoxy-phenyl}-propanoate de méthyle sont obtenus sous forme d'une huile incolore.

### f- acide 3-{4-[3-(4-benzyloxy-3-methoxy-phenyl)-propyl]-3-butoxy-phenyl}-propanoique

0,84ml (0,84 mmoles) d'une solution aqueuse d'hydroxyde de lithium de concentration 1N sont additionnés sur une solution de 276mg (0,56 mmoles) de 3-{4-[3-(4-benzyloxy-3-methoxy-phenyl)-propyl]-3-butoxy-phenyl}-propanoate de méthyle dans 3ml de tétrahydrofurane. Le mélange réactionnel est agité à température ambiante pendant 12 heures. Le milieu réactionnel est évaporé à sec puis le résidu est repris dans l'eau et le milieu est acidifié par l'addition d'une solution d'acide acétique puis extrait avec de l'acétate d'éthyle. Les phases organiques sont rassemblées, lavées par une solution aqueuse saturée de chlorure de sodium, séchées sur sulfate de magnésium, filtrées, évaporées. Après recristallisation dans le cyclohexane et filtration, 205mg (77%) d' acide 3-{4-[3-(4-benzyloxy-3-methoxy-phenyl)-propyl]-3-butoxy-phenyl}-propanoique sont obtenus sous forme de cristaux blancs de point de fusion 92°C.
RMN (¹H, CDCl₃): 0,97 (t, J= 7,4 Hz, 3H); 1,48 (m, 2H); 1,75 (m, 2H); 1,87 (m, 2H); 2,59 (q, 2H); 2,67 (t, J=8,1Hz, 2H); 2,92 (t, J=8,1 Hz, 2H); 3,88 (s, 3H); 3,93 (t, J=6,3Hz, 2H); 5,12 (s, 2H); 6,65-6,70 (m, 2H); 6,73 (d, J=6,3Hz, 1H); 7,02 (d, J=7,5Hz); 7,28-7,44 (m, 5H);

### Exemple 2 : acide 3-{3-butoxy-4-[3-(4-ethoxy-3-methoxy-phenyl)-propyl]-phenyl}-propanoique

### a- 3-{4-[3-(4-éthoxy-3-methoxy-phenyl)-propyl]-3-butoxy-phenyl}-propanoate de méthyle

0,21g (0,75 mmole) de carbonate de potassium puis 0,12ml (0,75 mmole) d'iodoéthane sont ajoutés sur une solution de 0,25 g (0,62 mmole) de 3-{3-butoxy-4-[3-(4-hydroxy-3-methoxy-phenyl)-propyl]-phenyl}-propanoate de méthyle (préparé selon l'exemple 1 d) dans 25ml de méthyléthylcétone. Le mélange réactionnel est chauffé à 70°C pendant 24 heures. Le milieu réactionnel est refroidi, dilué à l'acétate d'éthyle, lavé avec une solution aqueuse saturée de chlorure de sodium. La phase organique est séchée sur sulfate de sodium, filtrée, évaporée puis le résidu est purifié par chromatographie sur colonne de silice élué avec un mélange heptane/acétate d'éthyle 80/20. 240mg (93%) de 3-{4-[3-(4-éthoxy-3-methoxy-phenyl)-propyl]-3-butoxy-phenyl}-propanoate de méthyle sont obtenus sous forme d'une huile incolore sont obtenus.

### b- acide 3-{3-butoxy-4-[3-(4-ethoxy-3-methoxy-phenyl)-propyl]-phenyl}-propanoique

1,1ml (1,1 mmoles) d'une solution aqueuse d'hydroxyde de lithium de concentration 1 N sont additionnés sur une solution de 240mg (0,56 mmoles) de 3-{3-butoxy-4-[3-(4-ethoxy-3-methoxy-phenyl)-propyl]-phenyl}-propanoate de méthyle dans 3ml de tétrahydrofurane. Le mélange réactionnel est agité à température ambiante pendant 12 heures. Le milieu réactionnel est concentré puis repris dans l'eau et acidifié par l'addition d'une solution aqueuse d'acide chlorhydrique puis extrait avec de l'acétate d'éthyle. Les phases organiques sont rassemblées, lavées par une solution aqueuse saturée de chlorure de sodium puis séchées sur sulfate de magnésium, filtrées. Les solvants sont évaporés. Après recristallisation du résidu obtenu dans 6ml de cyclohexane, 184mg (79%) d'acide 3-{3-butoxy-4-[3-(4-éthoxy-3-methoxy-phenyl)-propyl]-phenyl}-propanoique sont obtenus sous forme d'un solide blanc de point de fusion 66°C.
RMN (¹H, CDCl₃): 0,97 (t, J=7,4Hz, 3H); 1,44 (t, J=7Hz, 3H); 1,49 (m, 2H); 1,75 (m, 2H); 1,88 (m, 2H); 2,58-2,69 (m, 6H); 2,92 (t, J=7,6Hz, 2H); 3,85 (s, 3H); 3,94 (t, J=6,3Hz, 2H); 4,07 (q, J=7Hz, 2H); 6,67-6,71 (m, 4H); 6,78 (m, 1H); 7,03 (d, J=7,5Hz).

### Exemple 3 : acide 3-{3-butoxy-4-[3-(4-butoxy-3-methoxy-phenyl)-propyl]-phenyl}-propanoique

### a- 3-{4-[3-(4-butoxy-3-méthoxy-phenyl)-propyl]-3-propoxy-phenyl}-propanoate de méthyle

0,21g (0,75 mmole) de carbonate de potassium puis 0,85ml (0,75 mmole) de bromure de benzyle sont ajoutés sur une solution de 0,25g (0,62 mmole) de 3-{3-butoxy-4-[3-(4-hydroxy-3-methoxy-phenyl)-propyl]-phenyl}-propanoate de méthyle (préparé selon l'exemple 1 d) dans 25ml de méthyléthylcétone. Le mélange réactionnel est chauffé à 70°C pendant 24 heures puis refroidi, dilué à l'acétate d'éthyle et lavé avec une solution aqueuse saturée de chlorure de sodium. La phase organique est séchée sur sulfate de sodium, filtrée, évaporée. Le résidu est purifié par chromatographie sur colonne de silice élué avec un mélange heptane/acétate d'éthyle 80/20. 259mg (93%) de 3-{4-[3-(4-butoxy-3-méthoxy-phenyl)-propyl]-3-propoxy-phenyl}-propanoate de méthyle sont obtenus sous forme d'une huile incolore.

### b- acide 3-{3-butoxy-4-[3-(4-butoxy-3-methoxy-phenyl)-propyl]-phenyl}-propanoique

1,14ml (0,85 mmole) d'une solution aqueuse d'hydroxyde de lithium de concentration 1 N sont additionnés sur une solution de 259mg (0,56 mmoles) de 3-{3-butoxy-4-[3-(4-butoxy-3-methoxy-phenyl)-propyl]-phenyl}-propanoate de méthyle dans 3ml de tétrahydrofurane. Le mélange réactionnel est agité à température ambiante pendant 12 heures puis concentrée sous vide. Le résidu est repris dans l'eau, acidifié par l'addition d'une solution d'acide chlorhydrique de concentration 1 N puis extrait avec de l'acétate d'éthyle. Les phases organiques sont rassemblées, lavées par une solution aqueuse saturée de chlorure de sodium, séchées sur sulfate de magnésium, filtrées, évaporées. Après trituration dans l'heptane, 208mg (83%) d' acide 3-{3-butoxy-4-[3-(4-butoxy-3-methoxy-phenyl)-propyl]-phenyl}-propanoique sont obtenus sous forme de cristauux blancs de point de fusion 64°C.
RMN (¹H, CDCl₃): 0,96 (t, J=7,4Hz, 3H); 0,98 (t, J=7,5Hz, 3H); 1,45-1,51 (m, 4H); 1,74-1,88 (m, 6H); 2,57-2,69 (m, 6H); 2,92 (t, J=7,6Hz, 2H); 3,84 (s, 3H); 3,93 (t, J=6,3Hz, 2H); 3,99 (t, J=6,8Hz, 2H); 6,67-6,71 (m, 4H); 6,79 (m, 1H); 7,03 (d, J=7,5 Hz, 1H);

### Exemple 4 : acide 3-{3- butoxy-4-[3-(4-methanesulfonyloxy-3-methoxy-phenyl)-propyl]-phenyl}-propanoique

### a- méthanesulfonate de 4-al/yl-2-methoxy-phényle

3,1ml (40 mmoles) de chlorure de méthanesulfonyle sont additionnés à une solution de 6g (36 mmoles) d'eugénol, 5,5ml (43 mmoles) de triéthylamine dans 100ml de dichlorométhane, préalablement refroidie à -20°C. Après agitation à température ambiante pendant 3 heures, le milieu réactionnel est traité avec de l'eau et de l'acétate d'éthyle. La phase organique est séchée sur sulfate de magnésium, filtrée, évaporée. 9,5g (100%) de méthanesulfonate de 4-allyl-2-méthoxy-phényle sont obtenus.

### b- (E)-3-{3-butoxy-4-[(E)-3-(4-méthanesulfonyloxy-3-methoxy-phenyl)-propenyl]-phenyl}-acrylate de méthyle

Une solution de 334mg (1,4 mmole) de méthanesulfonate de 4-allyl-2-methoxy-phényle, 500mg (1,4 mmole) de (E)-3-(3-Butoxy-4-iodo-phenyl)-acrylate de méthyle (préparé selon l'exemple 1 b), 15mg de palladium acétate, 48mg de 2-(dicyclohexylphosphino)biphényle dans 5ml d'un mélange diméthylformamide / triéthylamine 6/1 est agitée pendant 3 heures à 80°C. Après addition d'eau et extraction avec de l'acétate d'éthyle, les phases organiques sont lavées avec une solution aqueuse saturée de chlorure de sodium et séchées sur sulfate de sodium. L'huile résiduelle est purifiée par chromatographie sur colonne de silice éluée avec un mélange heptane / acétate d'éthyle 8/2.

### c- 3-{3-butoxy-4-[3-(4-méthanesulfonyloxy-3-methoxy-phenyl)-propyl]-phenyl}-propanoate de méthyle

100mg de palladium sur charbon sont ajoutés sur une solution de (E)-3-{3-butoxy-4-[(E)-3-(4-méthanesulfonyloxy-3-methoxy-phenyl)-propenyl]-phenyl}-acrylate de méthyle précédemment obtenu dans 10ml de méthanol. Le milieu réactionnel est agité pendant une heure à température ambiante sous une atmosphère d'hydrogène. Après filtration du catalyseur sur célite et évaporation du solvant, 600mg (91% pour les 2 étapes) de 3-{3-butoxy-4-[3-(4-méthanesulfonyloxy-3-methoxy-phenyl)-propyl]-phenyl}-propanoate de méthyle sont obtenus sous la forme d'une huile incolore.

### d- acide 3-{3-butoxy-4-[3-(4-méthanesulfonyloxy-3-méthoxy-phenyl)-propyl]-phenyl}-propanoique

600mg (1,25 mmole) de 3-{3-butoxy-4-[3-(4-méthanesulfonyloxy-3-methoxy-phenyl)-propyl]-phenyl}-propanoate de méthyle sont dissous dans 10ml de tétrahydrofuranne puis 1,8ml (1,8 mmole) d'une solution aqueuse d'hydroxyde de lithium de concentration 1 N sont ajoutés. Le milieu réactionnel est agité pendant 15 heures à température ambiante. Après addition d'eau et acidification avec de l'acide acétique, le milieu réactionnel est extrait avec de l'acétate d'éthyle. Les phases organiques sont rassemblées, séchées sur sulfate de sodium, filtrées, évaporées. Le résidu obtenu est purifié par chromatographie sur colonne de silice élué avec un mélange heptane / acétate d'éthyle 8/2. 480mg (82%) d'acide 3-{3-butoxy-4-[3-(4-méthanesulfonyloxy-3-méthoxy-phenyl)-propyl]-phenyl}-propanoique sont obtenus après cristallisation dans un mélange éther isopropylique / pentane 2/8.
RMN (¹H, CDCl₃): 0,97 (t, J=7,4 Hz, 3H); 1,48 (m, 2H); 1,75 (m, 2H); 1,90 (m, 2H); 2,61-2,69 (m, 6H); 2,92 (t, J=7,6 Hz, 2H); 3,16 (s, 3H); 3,86 (s, 3H); 3,94 (t, J=6,4 Hz, 2H); 6,78 (s, 1H); 6,70 (d, J=7,6 Hz, 1H); 6,77-6,80 (m, 2H); 7,03 (d, J=7,6 Hz, 1H); 7,19 (d, J=8,6 Hz, 1H).

### Exemple 5 : acide 3-(4-{3-[4-(butane-1-sulfonyloxy)-3-méthoxy-phenyl]-propyl}-3-butoxy-phenyl)-propanoique

### a- (E)-3-{3-butoxy-4-[(E)-3-(4-hydroxy-3-méthoxy-phenyl)-propenyl]-phenyl}-acrylate de méthyle

Une solution de 1,1g (12,1 mmole) d'eugénol, 2,0g (5,5 mmoles) de (E)-3-(3-butoxy-4-iodo-phenyl)-acrylate de méthyle (préparé selon l'exemple 1b), 24mg (0,1 mmole) de palladium (II) acétate, 77mg (0,2 mmole) de 2-(dicyclohexylphosphino)biphényle dans 15ml d'un mélange diméthylformamide/ triéthylamine 6/1 est agité à 90°C pendant 3 heures. Après addition d'eau, le milieu réactionnel est extrait avec de l'acétate d'éthyle. Les phases organiques sont lavées avec une solution aqueuse saturée de chlorure de sodium, séchées sur sulfate de sodium, filtrées, évaporées. L'huile résiduelle est purifiée par chromatographie sur colonne de silice éluée avec un mélange heptane / acétate d'éthyle 8/2.

### b- 3-{3-butoxy-4-[3-(4-hydroxy-3-méthoxy-phenyl)-propyl]-phenyl}-propanoate de méthyle

500mg de palladium sur charbon sont ajoutés sur une solution de (E)-3-{3-butoxy-4-[(E)-3-(4-hydroxy-3-méthoxy-phenyl)-propenyl]-phenyl}-acrylate de méthyle dans 25ml de méthanol. Le milieu réactionnel est agité sous une atmosphère d'hydrogène, à température ambiante pendant 3 heures. Après filtration du catalyseur et évaporation du solvant, 2,0g (90%) de 3-{3-butoxy-4-[3-(4-hydroxy-3-méthoxy-phenyl)-propyl]-phenyl}-propanoate de méthyle sont obtenus sous forme d'une huile incolore.

### c- 3-(4-{3-[4-(butane-1-sulfonyloxy)-3-méthoxy-phenyl]-propyl}-3-butoxy-phenyl)-propanoate de méthyle

Une solution de 400mg (1 mmole) de 3-{3-butoxy-4-[3-(4-hydroxy-3-méthoxy-phenyl)-propyl]-phenyl}-propanoate de méthyle, de 170µl (1,3 mmole) de chlorure de butane sulfonyle, 250µl de triéthylamine dans 10ml de tétrahydrofuranne est agité pendant 12 heures à température ambiante. Après addition d'eau et extraction avec de l'acétate d'éthyle, les phases organiques sont lavées avec une solution aqueuse saturée de chlorure de sodium, séchées sur sulfate de sodium, filtrées, évaporées. L'huile résiduelle est purifiée par chromatographie sur colonne de silice éluée avec un mélange heptane / acétate d'éthyle 8/2. 410mg de 3-(4-{3-[4-(butane-1-sulfonyloxy)-3-méthoxy-phenyl]-propyl}-3-butoxy-phenyl)-propanoate de méthyle sont obtenus sous forme d'une huile jaune.

### d- acide 3-(4-{3-[4-(butane-1-sulfonyloxy)-3-méthoxy-pheny/]-propyl}-3-butoxy-phenyl)-propanoique

410mg de 3-(4-{3-[4-(butane-1-sulfonyloxy)-3-méthoxy-phenyl]-propyl}-3-butoxy-phenyl)-propanoate de méthyle sont dissous dans 10ml de tétrahydrofurane et 1,8ml d'une solution aqueuse d'hydroxyde de lithium de concentration 1N est ajoutée. Le milieu réactionnel est agité pendant 15 heures à température ambiante. Après addition d'eau et acidification à pH 4 avec de l'acide acétique, le milieu réactionnel est extrait à l'acétate d'éthyle. La phase organique est séchée sur sulfate de sodium, filtrée, évaporée. Le résidu obtenu est purifié par chromatographie sur colonne de silice élué avec un mélange heptane / acétate d'éthyle 8/2. 136 mg (27% pour les deux étapes) d'acide 3-(4-{3-[4-(butane-1-sulfonyloxy)-3-méthoxy-phenyl]-propyl}-3-butoxy-phenyl)-propanoique sont obtenus sous forme d'une huile incolore.
RMN (¹H, CDCl₃): 0,98 (t, J=7,4 Hz, 3H); 1,01 (t, J=7 Hz, 3H); 1,49-1,54 (m, 4H); 1,77 (m, 2H); 1,91 (m, 2H); 1,99 (m, 2H); 2,63-2,67 (m, 6H); 2,92 (t, J=7,6 Hz, 2H);
3,28 (t, J=6,4 Hz, 2H); 3,70 (s, 3H); 4,14 (m, 2H); 6,69 (s, 1H); 6,71 (d, J=7,5 Hz, 2H); 6,79 (m, 2H); 7,04 (dd, J=2,8 Hz, J=7,4 Hz, 1H); 7,20 (dd, J=1,7 Hz, J=7 Hz, 1H).

### Exemple 6 : acide 3-{3-butoxy-4-[3-(4-ethanesulfonyloxy-3-methoxy-phenyl)-propyl]-phenyl)-propanoique

### a- 3-{3-butoxy-4-[3-(4-éthanesulfonyloxy-3-méthoxy-phenyl)-propyl]-phenyl}-propanoate de méthyle

0,2ml (1,6 mmole) de triéthylamine puis 0,15ml (1,7 mmole) de chlorure d'éthane sulfonyle sont ajoutés sur une solution de 0,5g (1,25 mmole) de 3-{3-butoxy-4-[3-(4-hydroxy-3-méthoxy-phenyl)-propyl]-phenyl}-propanoate de méthyle (préparé selon l'exemple 1 d) dans 3ml de dichlorométhane, préalablement refroidie à -20°C. Le milieu réactionnel est agité à température ambiante pendant 3 heures puis la réaction est traitée par l'addition d'une solution aqueuse saturée de chlorure de sodium et extraite à l'acétate d'éthyle. Les phases organiques sont rassemblées, séchées sur sulfate de sodium, filtrées et évaporées. Le résidu est purifié par chromatographie sur colonne de silice élué avec un mélange heptane/acétate d'éthyle 90/10. 618mg (99%) de 3-{3-butoxy-4-[3-(4-éthanesulfonyloxy-3-méthoxy-phenyl)-propyl]-phenyl}-propanoate de méthyle sont obtenus sous forme d'une huile incolore.

### b- acide 3-{3-butoxy-4-[3-(4-éthanesulfonyloxy-3-méthoxy-phenyl)-propyl]-phenyl)-propanoique

1,1ml (0,85 mmole) d'une solution aqueuse d'hydroxyde de lithium de concentration 1 N sont additionnés sur une solution de 259mg (0,6 mmoles) de 3-{3-butoxy-4-[3-(4-éthanesulfonyloxy-3-méthoxy-phenyl)-propyl]-phenyl}-propanoate de méthyle dans 3ml de tétrahydrofurane. Le mélange réactionnel est agité à température ambiante pendant 12 heures, concentré puis repris dans l'eau, acidifié par l'addition d'une solution d'acide chlorhydrique puis extrait avec de l'acétate d'éthyle. Les phases organiques sont rassemblées, lavées par une solution saturée de chlorure de sodium, séchées sur sulfate de magnésium, filtrées, évaporées. Après trituration dans l'heptane, 208mg (83%) d' acide 3-{3-butoxy-4-[3-(4-éthanesulfonyloxy-3-methoxy-phenyl)-propyl]-phenyl)-propanoique sous forme de cristaux blancs de point de fusion 64°C.
RMN (¹H, CDCl₃):0,98 (t, J=7,4Hz, 3H); 1,46 (m, 2H); 1,53 (t, J=7,4Hz, 3H); 1,77 (m, 2H); 1,90 (m, 2H); 2,60-2,69 (m, 6H); 2,92 (t, J=7,6Hz, 2H); 3,29 (q, J=7,4Hz, 2H); 3,85 (s, 3H); 3,93 (t, J=6,3Hz, 2H); 6,66-6,71 (m, 2H); 6,76-6,78 (m, 2H); 7,02 (d, J=7,5Hz, 1H); 7,19 (d, J=8,7Hz, 1H).

### Exemple 7 : acide 3-[4-{3-[4-(butane-1-sulfonyloxy)-3-methoxy-phenyl]-propyl}-3-(3-fluoro-benzyloxy)-phenyl]-propanoique

### a- 3-benzyloxy-4-iodo-benzaldehyde

47ml (411 mmoles) de chlorure de benzyle sont ajoutés sur une solution de 93g (374 mmoles) de 3-hydroxy-4-iodo-benzaldéhyde dans 600ml de méthyléthylcétone en présence de 103g (748 mmoles) de carbonate de potassium. Le milieu réactionnel est chauffé à 78°C pendant 18 heures. Le solide est filtré et le solvant est évaporé. Le solide obtenu est lavé à l'heptane et 114g (90%) de 3-benzyloxy-4-iodo-benzaldéhyde sous forme de cristaux blancs sont obtenus.

### b- (E)-3-(3-benzyloxy-4-iodo-phenyl)-acrylate de méthyle

170g (506 mmoles) de (triphénylphosphoranylidène) acétate de méthyle sont ajoutés sur une solution de 114g (337 mmoles) de 3-benzyloxy-4-iodo-benzaldéhyde dans 570ml de toluène. Le mélange réactionnel est chauffé à reflux pendant 2 heures. Le solvant est évaporé et l'huile obtenue est purifiée par chromatographie sur colonne de silice éluée avec un mélange heptane/dichlorométhane 50/50. 115g (87%) de (E)-3-(3-benzyloxy-4-iodo-phenyl)-acrylate de méthyle sont obtenus sous forme de cristaux jaune pâle.

### c- (4-Allyl-2-méthoxy-phenyl)-méthanesulfonate de butyle

5,2ml (40 mmoles) de chlorure de butanesulfonyle sont additionnés à une solution de 6g (36 mmoles) d'eugénol, 5,5ml (43 mmoles) de triéthylamine dans 100ml de dichlorométhane, préalablement refroidie à -20°C. Après agitation à température ambiante pendant 3 heures, le milieu réactionnel est traité avec de l'eau et de l'acétate d'éthyle. La phase organique est séchée sur sulfate de magnésium, filtrée, évaporée. 10,8g (100%) de (4-allyl-2-méthoxyphényl)-méthanesulfonate de butyle sont obtenus.

### d- (E)-3-(3-benzyloxy-4-{(E)-3-[4-(butane-1-sulfonyloxy)-3-méthoxy-phenyl]-propenyl}-phenyl)-acrylate de méthyle

0,3g (0,8 mmole) de 2-(dicyclohexylphosphino)biphényle, 0,1g (0,4 mmole) d'acétate de palladium (II) puis 3,4ml (25mmoles) de triéthylamine sont ajoutés sur une solution de 7g (3 mmoles) de (4-allyl-2-méthoxy-phényl)-méthanesulfonate de butyle et 8,1g (20 mmoles) de (E)-3-(3-benzyloxy-4-iodo-phényl)-acrylate de méthyle dans 80ml de diméthylformamide. Le mélange réactionnel est chauffé à 90°C pendant 15 heures. Après addition de 20ml d'eau puis extraction avec de l'acétate d'éthyle, les phases organiques sont rassemblées, lavées avec une solution saturée de chlorure de sodium, séchées sur sulfate de sodium, filtrées, évaporées. 11g (100%) de 4-{(E)-3-[4-(butane-1-sulfonyloxy)-3-méthoxy-phenyl]-propenyl}-3-butoxy-benzoate de méthyle sont obtenus.

### e- 3-(4-{3-[4-(butane-1-sulfonyloxy)-3-méthoxy-phenyl]-propyl}-3-hydroxy-phenyl)-propanoate de méthyle

11g (20 mmoles) de (E)-3-(3-benzyloxy-4-{(E)-3-[4-(butane-1-sulfonyloxy)-3-méthoxyphenyl]-propenyl}-phenyl)-acrylate de méthyle sont dissous dans 100ml de méthanol puis 1,1g (10% massique) de palladium sur charbon est ajouté. Le milieu réactionnel est placé sous 1 atmosphère d'hydrogène pendant 24 heures puis filtré sur célite, rincé au dichlorométhane et les jus de filtration sont concentrés. Le résidu est purifié par chromatographie sur colonne de silice élué avec un mélange heptane / acétate d'éthyle 8/2. 8g (73%) de 3-(4-{3-[4-(butane-1-sulfonyloxy)-3-méthoxy-phényl]-propyl}-3-hydroxy-phenyl)-propanoate de méthyle sont obtenus.

### f- 3-[4-{3-[4-(butane-1-sulfonyloxy)-3-méthoxy-phenyl]-propyl}-3-(3-fluoro-benzyloxy)-phenyl]-propanoate de méthyle

0,2g (1,2 mmole) de carbonate de potassium suivi de 0,1ml (0,9 mmole) de bromure de 3-fluorobenzyle sont ajoutés à une solution de 0,4g (0,8 mmole) de 3-(4-{3-[4-(butane-1-sulfonyloxy)-3-méthoxy-phenyl]-propyl}-3-hydroxy-phenyl)-propanoate de méthyle. Le milieu réactionnel est agité à 80°C pendant 15 heures puis traité avec de l'eau et extrait à l'acétate d'éthyle. La phase organique est séchée sur sulfate de magnésium, filtrée et concentrée.

Le résidu obtenu est purifié par chromatographie sur colonne de silice élué avec un mélange heptane / acétate d'éthyle 8/2. 0,27g (60%) de 3-[4-{3-[4-(butane-1-sulfonyloxy)-3-méthoxyphenyl]-propyl}-3-(3-fluoro-benzyloxy)-phenyl]-propanoate de méthyle sont obtenus.

### g- acide 3-[4-{3-[4-(butane-1-sulfonyloxy)-3-méthoxy-phenyl]-propyl}-3-(3-fluoro-benzyloxy)-phenyl]-propanoique

0,4g (1 mmole) d'hydroxyde de lithium monohydrate sont additionnés sur une solution de 0,3g (0,5 mmole) de 3-[4-{3-[4-(butane-1-sulfonyloxy)-3-méthoxy-phenyl]-propyl}-3-(3-fluorobenzyloxy)-phenyl]-propanoate de méthyle correspondant dans 10ml d'un mélange tétrahydrofurane / méthanol / eau (5/1/1). Le milieu réactionnel est agité à température ambiante pendant 15 heures, traité avec de l'eau, acidifié à pH4 avec de l'acide acétique, extrait à l'acétate d'éthyle. La phase organique est séchée sur sulfate de magnésium, filtrée et concentrée. Le résidu obtenu est purifié par chromatographie sur colonne de silice élué avec un mélange dichlorométhane / méthanol 97/3. 0,23g (77%) d'acide 3-[4-{3-[4-(butane-1-sulfonyloxy)-3-méthoxy-phenyl]-propyl}-3-(3-fluoro-benzyloxy)-phényl]-propanoique sont obtenus sous forme d'une huile incolore.
RMN (¹H, CDCl₃): 0,89 (t, J=7,3Hz, 3H); 1,40 (m, 2H); 1,83-1,93 (m, 4H); 2,55-2,63 (m, 6H); 2,84 (t, J=7,6Hz, 2H); 3,18 (m, 2H); 3,74 (s, 3H); 4,97 (s, 2H); 6,66-6,69 (m, 4H); 6,93 (m, 1H); 7,00 (d, J=7,5Hz, 1H); 7,06-7,17 (m, 3H); 7,26 (m, 1H).

### Exemple 8 : acide 3-[4-{3-[4-(butane-1-sulfonyloxy)-3-méthoxy-phenyl]-propyl} -3-(4-fluoro-benzyloxy)-phenyl]-propanoique

### a- 3-[4-{3-[4-(butane-1-sulfonyloxy)-3-méthoxy-phenyl]-propyl}-3-(4-fluoro-benzyloxy)-phenyl]-propanoate de méthyle

0,2g (1,2 mmole) de carbonate de potassium suivi de 0,1ml (0,9 mmole) de bromure de 4-fluorobenzyle sont ajoutés à une solution de 0,4g (0,8 mmole) de 3-(4-{3-[4-(butane-1-sulfonyloxy)-3-méthoxy-phenyl]-propyl}-3-hydroxy-phenyl)-propanoate de méthyle (préparé selon l'exemple 7e). Le milieu réactionnel est agité à 80°C pendant15 heures puis traité avec de l'eau et extrait à l'acétate d'éthyle. La phase organique est séchée sur sulfate de magnésium, filtrée et concentrée.
Le résidu obtenu est purifié par chromatographie sur colonne de silice élué avec un mélange heptane / acétate d'éthyle 8/2. 0,3g (65%) de 3-[4-{3-[4-(butane-1-sulfonyloxy)-3-méthoxyphenyl]-propyl}-3-(4-fluoro-benzyloxy)-phenyl]-propanoate de méthyle sont obtenus.

### b- acide 3-[4-{3-[4-(butane-1-sulfonyloxy)-3-méthoxy-phenyl]-propyl} -3-(4-fluoro-benzyloxy)-phenyl]-propanoique

0,4g (1 mmole) d'hydroxyde de lithium monohydrate sont additionnés sur une solution de 0,3g (0,5 mmole) de 3-[4-{3-[4-(butane-1-sulfonyloxy)-3-méthoxy-phenyl]-propyl}-3-(4-fluorobenzyloxy)-phenyl]-propanoate de méthyle correspondant dans 10ml d'un mélange tétrahydrofurane / méthanol / eau (5/1/1). Le milieu réactionnel est agité à température ambiante pendant 15 heures, traité avec de l'eau, acidifié à pH4 avec de l'acide acétique, extrait à l'acétate d'éthyle. La phase organique est séchée sur sulfate de magnésium, filtrée et concentrée. Le résidu obtenu est purifié par chromatographie sur colonne de silice élué avec un mélange dichlorométhane / méthanol 97/3. 0,24g (85%) d'acide 3-[4-{3-[4-(butane-1-sulfonyloxy)-3-méthoxy-phenyl]-propyl}-3-(4-fluoro-benzyloxy)-phenyl]-propanoique sont obtenus sous forme d'une huile incolore.
RMN (¹H, CDCl₃): 0,89 (t, J=7,4Hz, 3H); 1,43 (m, 2H); 1,81-1,92 (m, 4H); 2,53-2,61 (m, 6H); 2,84 (t, J=7,6Hz, 1H); 3,19 (m, 2H); 3,73 (s, 3H); 4,93 (s, 2H); 6,64-6,69 (m, 4H); 6,97-7,01 (m, 3H); 7,08 (d, J=8 Hz, 1H); 7,18-7,31 (m, 2H).

### Exemple 9 : acide 3-(4-{3-[4-(butane-1-sulfonyloxy)-3-méthoxy-phenyl]-propyl}- 3-cyclopropylméthoxy-phenyl)-propanoique

### a- 3-(4-{3-[4-(butane-1-sulfonyloxy)-3-méthoxy-phenyl]-propyl}- 3-cyclopropylméthoxyphenyl)-propanoate de méthyle

0,2g (1,2 mmole) de carbonate de potassium suivi de 0,12g (0,9 mmole) de bromométhylcyclopropane sont ajoutés à une solution de 0,4g (0,8 mmole) de 3-(4-{3-[4-(butane-1-sulfonyloxy)-3-méthoxy-phenyl]-propyl}-3-hydroxy-phenyl)-propanoate de méthyle (préparé selon l'exemple 7e). Le milieu réactionnel est agité à 80°C pendant15 heures puis traité avec de l'eau et extrait à l'acétate d'éthyle. La phase organique est séchée sur sulfate de magnésium, filtrée et concentrée.
Le résidu obtenu est purifié par chromatographie sur colonne de silice élué avec un mélange heptane / acétate d'éthyle 8/2. 0,26g (61%) de 3-(4-{3-[4-(butane-1-sulfonyloxy)-3-méthoxyphenyl]-propyl}- 3-cyclopropylméthoxy-phenyl)-propanoate de méthyle sont obtenus.

### b- acide 3-(4-{3-[4-(butane-1-sulfonyloxy)-3-méthoxy-phenyl]-propyl}- 3-cyclopropylméthoxyphenyl)-propanoique

0,4g (1 mmole) d'hydroxyde de lithium monohydrate sont additionnés sur une solution de 0,26g (0,5 mmole) de 3-(4-{3-[4-(butane-1-sulfonyloxy)-3-méthoxy-phenyl]-propyl}- 3-cyclopropylméthoxy-phenyl)-propanoate de méthyle correspondant dans 10ml d'un mélange tétrahydrofurane / méthanol / eau (5/1/1). Le milieu réactionnel est agité à température ambiante pendant 15 heures, traité avec de l'eau, acidifié à pH4 avec de l'acide acétique, extrait à l'acétate d'éthyle. La phase organique est séchée sur sulfate de magnésium, filtrée et concentrée. Le résidu obtenu est purifié par chromatographie sur colonne de silice élué avec un mélange dichlorométhane / méthanol 97/3. 0,24g (100%) d'acide 3-(4-{3-[4-(butane-1-sulfonyloxy)-3-méthoxy-phenyl]-propyl}-3-cyclopropylméthoxy-phényl)-propanoique sont obtenus sous forme d'une huile incolore.
RMN (¹H, CDCl₃): 0,25 (m, 2H); 0,52 (m, 2H); 0,90 (t, J=7,3Hz, 3H); 1,17 (m, 1H); 1,42 (m, 2H); 2,56-2,61 (m, 6H); 2,83 (t, J=7,5 Hz, 2H); 3,72 (d, J=6,6 Hz, 2H); 3,78 (s, 3H); 6,58 (s, 1H); 6,63 (dd, J=1,4Hz, J=7,6 Hz, 1H); 6,71-6,73 (m, 2H); 6,96 (d, J=7,6Hz, 1H); 7,11 (d, J=8,6Hz, 1H).

### Exemple 10 : acide 3-[4-{3-[4-(butane-1-sulfonyloxy)-3-methoxy-phenyl]-propyl}-2-(3-methoxy-benzyloxy)-phenyl]-propanoique

### a- acide 3-hydroxy-4-iodo benzoique

1319g (1,52 moles) d'une solution commerciale d'hypochlorite de sodium 9,6% sont additionnés goutte à goutte à une solution de 200g (1,45 moles) d'acide 3-hydroxybenzoique, 61g (1,52 moles) d'hydroxyde de sodium en poudre, 228g (1,52 moles) d'iodure de sodium dans 2I de méthanol préalablement refroidie à 0°C. Le milieu réactionnel est agité à température ambiante pendant 72 heures. Après évaporation du méthanol, la solution est refroidie à 10°C et acidifiée avec une solution aqueuse d'acide chlorhydrique jusqu'à pH2. Le mélange est agité pendant 2 heures et le produit précipite. Le produit est filtré, abondamment lavé à l'eau et séché sous vide à 50°C.150g (39%) d' acide 3-hydroxy-4-iodo benzoique sont obtenus sous forme d'un solide blanc.

### b- 3-hydroxy-4-iodo-benzoate de méthyle

Une solution de 140g (530 mmoles) d'acide 3-hydroxy-4-iodo benzoique et de 20,2g (110 mmoles) d'acide para toluènesulfonique dans 900ml de méthanol est chauffée à reflux pendant 18 heures. Après refroidissement, 700ml d'eau sont additionnés et le milieu est agité pendant 18 heures. Le produit précipite et est filtré. Après séchage sous vide à 50°C, 137g (93%) de 3-hydroxy-4-iodo-benzoate de méthyle sont obtenus sous forme d'un solide blanc.

### c-3-benzyl-4-iodobenzoate de méthyle

47ml ( 411 mmoles) de chlorure de benzyle sont additionnés à une solution de 104g (374 mmoles) de 3-hydroxy-4-iodo-benzoate de méthyle et de 103g (748 mmoles) de carbonate de potassium dans 600ml de méthyl-éthylcétone puis le milieu réactionnel est chauffé à reflux pendant 8 heures. Après refoidissement, le milieu réactionnel est filtré, le précipité est rincé à l'acétate d'éthyle et le filtrat est évaporé à sec. Le résidu est repris dans un mélange d'eau et d'acétate d'éthyle. La phase organique est séchée sur sulfate de sodium, filtrée, évaporée. 139g (100%) de 3-benzyl-4-iodobenzoate de méthyle sont obtenus sous forme d'un solide blanc.

### d- alcool 3-benzyloxy-4-iodobenzylique

Une solution de 139g (374 mmoles) de 3-benzyl-4-iodobenzoate de méthyle dans 550ml de tétrahydrofurane est additionnée goutte à goutte à une solution de 12,9g (563 mmoles) de borohydrure de lithium dans 150ml de tétrahydrofurane puis le milieu réactionnel est chauffé à reflux pendant 3 heures. Après refroidissement, 300ml d'une solution aqueuse saturée de chlorure d'ammonium sont additionnés et le milieu réactionnel est extrait à l'acétate d'éthyle. La phase organique est lavée à l'eau, séchée sur sulfate de sodium, filtrée, évaporée. 125g (97%) d'alcool 3-benzyloxy-4-iodobenzylique sont obtenus sous forme de cristaux blancs.

### e- 3-benzyloxy-4-iodobenzaldéhyde

Une solution de 125g (370 mmoles) d'alcool 3-benzyloxy-4-iodobenzylique, 160g (1,84 moles) de dioxyde de manganèse dans 750ml de dichlorométhane est agitée à température ambiante pendant 18 heures. La réaction n'étant pas totale, 160g (1,84 moles) de dioxyde de manganèse sont à nouveau ajoutés et le milieu est agité pendant 6 heures. Le milieu réactionnel est filtré sur célite puis le filtrat est concentré sous vide. 114g (92%) de 3-benzyloxy-4-iodobenzaldéhyde sont obtenus sous forme d'une huile jaune.

### f- (E)-3-(2-benzyloxy-4-iodo-phenyl)-acrylate de méthyle

170g (506 mmoles) de triphénylphosphoranylidène acétate de méthyle sont ajoutés par portions à une solution de 114g (337 mmoles) de 3-benzyloxy-4-iodobenzaldéhyde dans 570ml de toluène et le milieu réactionnel est chauffé à reflux pendant deux heures. Après refroidissement, le milieu réactionnel est filtré sur célite puis concentré sous vide. Le résidu obtenu est purifié par chromatographie sur colonne de silice élué avec un mélange heptane / acétate d'éthyle 80/20. 113g (85%) de (E)-3-(2-benzyloxy-4-iodo-phenyl)-acrylate de méthyle sont obtenus sous forme d'une poudre jaune.

### g- 1-butane sulfonate de 4-allyl-2-méthoxy-phényle

13ml (0,1 mole) de chlorure de butane sulfonyle sont additionnés goutte à goutte sur une solution de 15g (0,09 mole) d'eugénol, de 16ml (0,11 mole) de triéthylamine dans 150ml de dichlorométhane préalablement refroidie à -20°C. Le mélange réactionnel est agité pendant

quatre heures à température ambiante. La réaction est traitée par addition de 50ml d'eau et extraction au dichlorométhane. La phase organique est séchée sur sulfate de sodium, filtrée, évaporée. Le résidu est purifié par chromatographie sur colonne de silice élué avec un mélange heptane / acétate d'éthyle 90/10. 24,3g (74%) de 1-butane-1-sulfonate de 4-allyl-2-methoxy-phenyle sous forme d'une huile jaune.

### h- (E)-3-(2-benzy/oxy-4-{(E)-3-[4-(butane-1-su/fony/oxy)-3-methoxy-phenyl]-propenyl}-phenyl)-acrylate de méthyle

71 mg (0,2 mmole) de 2-(dicyclohexylphosphino)biphenyle et 23mg (0,1 mmole) d'acétate de palladium sont additionnés à une solution de 2g (5,07 mmoles) de (E)-3-(2-benzyloxy-4-iodo-phenyl)-acrylate de méthyle, 1,1ml (7,6 mmoles) de triéthylamine, 2,9g (5,07 mmoles) de 1-butane sulfonate de 4-allyl-2-méthoxy-phényle dans 20ml de diméthylformamide. Le mélange réactionnel est chauffé à 80°C pendant quatre heures. La réaction est traitée par l'addition de 50ml d'eau puis extraite avec de l'acétate d'éthyle. Les phases organiques sont rassemblées, lavées avec une solution saturée de chlorure de sodium, séchées sur sulfate de sodium, filtrées puis les solvants sont évaporés. Le résidu est purifié par chromatographie sur colonne de silice élué avec un mélange heptane/acétate d'éthyle 90/10. 2,53g (91%) de (E)-3-(2-benzyloxy-4-{(E)-3-[4-(butane-1-sulfonyloxy)-3-methoxy-phenyl]-propenyl}-phenyl)-acrylate de méthyle sont obtenus sous forme d'une huile jaune.

### i- 3-(4-{3-[4-(butane-1-sulfonyloxy)-3-methoxy-phenyl]-propyl}-2-hydroxy-phenyl)-propanoate de méthyle

78mg (10% équivalent massique) de palladium sur charbon 10% sont ajoutés sur une solution de 780mg (1,4 mmole) de (E)-3-(2-benzyloxy-4-{(E)-3-[4-(butane-1-sulfonyloxy)-3-methoxy-phenyl]-propenyl}-phenyl)-acrylate de méthyle dans 8ml de méthanol. Le mélange réactionnel est placé sous pression atmosphérique d'hydrogène à température ambiante pendant 16 heures puis filtré sur célite et rincé au dichloromethane. Les solvants sont évaporés puis le résidu est purifié par chromatographie sur colonne de silice élué avec un mélange heptane / acétate d'éthyle 60/40. 610mg (94%) de 3-(4-{3-[4-(butane-1-sulfonyloxy)-3-methoxy-phenyl]-propyl}-2-hydroxy-phenyl)-propanoate de méthyle sont obtenus sous forme d'une huile incolore.

### j- 3-[4-{3-[4-(butane-1-suifonyloxy)-3-methoxy-phenyl]-propyl}-2-(3-methoxy-benzyloxy)-phenyl]-propanoate de méthyle

68µl (0,5 mmole) de chlorure de 3-méthoxybenzyle sont additionnés à une solution de 200mg (0,5 mmole) de 3-(4-{3-[4-(butane-1-sulfonyloxy)-3-methoxy-phenyl]-propyl}-2-hydroxy-phenyl)-propanoate de méthyle, 89mg (0,5 mmole) de carbonate de potassium dans 5ml de méthyl-éthylcétone puis le milieu réactionnel est chauffé à 70°C pendant 48h. Après refroidissement, de l'eau est ajoutée puis le milieu réactionnel est extrait à l'acétate d'éthyle. La phase organique est séchée sur sulfate de sodium, filtrée, évaporée. Le résidu obtenu est purifié par chromatographie sur colonne de silice élué avec un mélange heptane / acétate d'éthyle 7/3. 120mg (48%) de 3-[4-{3-[4-(butane-1-sulfonyloxy)-3-methoxy-phenyl]-propyl}-2-(3-methoxy-benzyloxy)-phenyl]-propanoate de méthyle sont obtenus.

### k- acide 3-[4-{3-[4-(butane-1-sulfonyloxy)-3-methoxy-phenyl]-propyl}-2-(3-methoxybenzyloxy)-phenyl]-propanoique

0,62ml (0,62 mmole) d'une solution d'hydroxyde de lithium de concentration 1M sont ajoutés sur une solution de 181,6mg (0,31 mmole) de 3-[4-{3-[4-(butane-1-sulfonyloxy)-3-methoxyphenyl]-propyl}-2-(3-methoxy-benzyloxy)-phenyl]-propanoate de méthyle dans 5ml de tétrahydrofuranne. Le mélange réactionnel est agité une nuit à température ambiante. La réaction est traitée par l'addition de 10ml d'eau, acidifiée avec de l'acide chlorhydrique puis extraite avec de l'acétate d'éthyle. Les phases organiques sont rassemblées, séchées sur sulfate de sodium. Après évaporation des solvants, le résidu est purifié par chromatographie sur colonne de silice élué avec un mélange dichlorométhane/méthanol 95/5. 162mg (92%) d'acide 3-[4-{3-[4-(butane-1-sulfonyloxy)-3-methoxy-phenyl]-propyl}-2-(3-methoxybenzyloxy)-phenyl]-propanoique sont obtenus sous forme d'une huile jaune.
RMN ¹H (δ, CDCl₃) : 0,90 (t, J=7,3 Hz, 3H) ; 1,42 (m, 2H) ; 1,85-1,93 (m, 4H) ; 2,52 (m, 4H) ; 2,91 (t, J=7,3 Hz, 2H) ; 3,2 (m, 2H) ; 3,73 (s, 3H) ; 3,78 (s, 3H) ; 4,99 (s, 2H) ; 6,65-6,69 (m, 3H) ; 6,77 (m, 1H) ; 6,93 (m, 1H) ; 7,12 (d, J= 8Hz, 1H) ; 7,19 (m, 1H) ; 7,22-7,24 (m, 2H).

### Exemple 11 : acide 3-(4-{3-[4-(butane-1-sulfonyloxy)-3-methoxy-phenyl]-propyl}-2-butoxy-phenyl)-propanoique

### a- 3-(4-{3-[4-(butane-1-sulfonyloxy)-3-methoxy-phenyl]-propyl}-2-butoxy-pheny/)-propanoate de méthyle

0,11 ml (0,95 mmole) d'iodobutane sont ajoutés sur une solution de 400mg (0,86 mmole) de 3-(4-{3-[4-(butane-1-sulfonyloxy)-3-methoxy-phenyl]-propyl}-2-hydroxy-phenyl)-propanoate de méthyle (préparé selon l'exemple 10i), 178mg (1,29 mmoles) de carbonate de potassium dans 10ml de méthyl-éthylcétone puis le mélange réactionnel est chauffé à 70°C pendant 18 heures. La réaction est traitée par l'addition de 20ml d'eau puis extraite avec de l'acétate d'éthyle. Les phases organiques sont rassemblées, séchées sur sulfate de sodium, filtrées, évaporées. Le résidu est purifié par chromatographie sur colonne de silice élué avec un mélange heptane/acétate d'éthyle 70/30. 179mg (40%) de 3-(4-{3-[4-(butane-1-sulfonyloxy)-3-methoxy-phenyl]-propyl}-2-butoxy-phenyl)-propanoate de méthyle sont obtenus sous forme d'une huile incolore.

### b- acide 3-(4-{3-[4-(butane-1-sulfonyloxy)-3-methoxy-phenyl]-propyl}-2-butoxy-phenyl)-propanoique

0,66ml (0,66 mmole) d'une solution aqueuse d'hydroxyde de lithium de concentration 1 M sont ajoutés sur une solution de 171 mg (0,33 mmole) de 3-(4-{3-[4-(butane-1-sulfonyloxy)-3-methoxy-phenyl]-propyl}-2-butoxy-phenyl)-propanoate de méthyle dans 3ml de tétrahydrofurane puis le mélange réactionnel est agité pendant 18 heures à température ambiante. Après évaporation à sec, le milieu réactionnel est repris dans 10ml d'eau et acidifié avec de l'acide acétique jusqu'à pH4 puis extrait avec de l'acétate d'éthyle. Les phases organiques sont rassemblées, séchées sur sulfate de sodium, et filtrées. Les solvants sont évaporés puis le résidu est purifié par chromatographie sur colonne de silice élué avec un mélange dichlorométhane / méthanol 99/1. 99mg (60%) d'acide 3-(4-{3-[4-(butane-1-sulfonyloxy)-3-methoxy-phenyl]-propyl}-2-butoxy-phenyl)-propanoique sont obtenus sous forme d'une huile jaune.
RMN ¹H (δ, CDCl₃) : 0,97 (t, J=7,3 Hz, 3H) ; 1 ,01 (t, J=7,3 Hz, 3H); 1,49-1,55 (m, 4H); 1,80 (m, 2H); 1,82-1,90 (m, 4H); 2,62-2,68 (m, 6H) ; 2,94 (t, J=7,5 Hz, 2H) ; 3,30 (t, J=7,8 Hz, 2H); 3,87 (s, 3H); 3,98 (t, J=6,3 Hz, 2H); 6,67 (s, 1H); 6,70 (d, J=7,6 Hz, 1H); 6,78-6,80 (m, 2H); 7,08 (d, J=7,5 Hz, 1H); 7,21 (d, J=8,7 Hz, 1H).

### Exemple 12 : TESTS DE TRANSACTIVATION PPARs EN COURBES CROISEES

L'activation des récepteurs PPAR par un agoniste (activateur) dans des cellules HeLN conduit à l'expression d'un gène reporter, la luciférase, qui, en présence d'un substrat génère de la lumière. La modulation des récepteurs PPAR est mesurée en quantifiant la luminescence produite après incubation des cellules en présence d'un agoniste de référence. Les ligands vont déplacer l'agoniste de son site. La mesure de l'activité se fait par la quantification de la lumière produite. Cette mesure permet de déterminer l'activité modulatrice des composés selon l'invention par la détermination de la constante qui représente l'affinité de la molécule pour le récepteur PPAR. Cette valeur pouvant fluctuer selon l'activité basale et l'expression du récepteur, on la dénomme Kd apparent (KdApp en nM).

Pour déterminer cette constante, des « courbes croisées » du produit à tester contre un agoniste de référence sont réalisées en plaque de 96 puits : 10 concentrations du produit à tester plus une concentration 0 sont disposées en ligne, et 7 concentrations de l'agoniste plus une concentration 0 sont disposées en colonne. Ceci représente 88 points de mesure pour 1 produit et 1 récepteur. Les 8 puits restants sont utilisés pour des contrôles de répétabilité.

Dans chaque puits, les cellules sont en contact avec une concentration du produit à tester et une concentration de l'agoniste de référence, l'acide 2-(4-{2-[3-(2,4-difluoro-phenyl)-1-heptylureido]-ethyl}-phenylsulfanyl)-2-methyl-propionique pour PPARα, l'acide {2-methyl-4-[4-methyl-2-(4-trifluoromethyl-phenyl)-thiazol-5-ylmethylsulfanyl]-phenoxy}-acetique pour PPARδ et le 5-{4-[2-(methyl-pyridin-2-yl-amino)-ethoxy]-benzyl}-thiazolidine-2,4-dione pour PPARγ. Des mesures sont également réalisées pour les témoins agonistes totaux avec les mêmes produits.

Les lignées cellulaires HeLN utilisées sont des transfectants stables contenant les plasmides ERE-βGlob-Luc-SV-Neo (gène reporter) et PPAR (α, δ, γ) Gal-hPPAR. Ces cellules sont ensemencées en plaques 96 puits à raison de 10 000 cellules par puits dans 100µl de milieu DMEM sans rouge de phénol et supplémenté par 10% de sérum de veau délipidé. Les plaques sont ensuite incubées à 37°C, 7% CO₂ pour 16 heures.

Les différentes dilutions des produits à tester et du ligand de référence sont rajoutées à raison de 5 µl par puits. Les plaques sont ensuite incubées 18 heures à 37°C, 7% CO₂.

Le milieu de culture est éliminé par retournement et 100 µl d'un mélange 1 :1 PBS/Luciferine est ajouté à chaque puits. Après 5 minutes, les plaques sont lues par le lecteur de luminescence.

Ces courbes croisées permettent de déterminer les AC50 (concentration à laquelle on observe 50% d'activation) du ligand de référence à différentes concentrations de produit à tester. Ces AC50 sont utilisées pour calculer la régression de Schild en traçant une droite répondant à l'équation de Schild *(*« quantitation in receptor pharmacology » Terry P.Kenakin, Receptors and Channels, 2001,7, 371-385) qui conduit à l'obtention des valeurs de Kd app (en nM).

Résultats de transactivation :

| Composés | PPAR alpha Kd app (nM) | PPARs delta Kd app (en nM) | PPAR gamma Kd app (en nM) |
|---|---|---|---|
| Référence 1 : acide 2-(4-{2-[3-(2,4-difluoro-phenyl)-1-heptyl-ureido]-ethyl}-phenylsulfanyl)-2-methyl-propionique | 200 | n.a. | n.a |
| Référence 2 : acide {2-methyl-4-[4-methyl-2-(4-trifluoromethyl-phenyl)-thiazol-5-ylmethylsulfanyl]-phenoxy}-acetique | n.a. | 10 | n.a |
| Référence 3 : 5-{4-[2-(methyl-pyridin-2-yl-amino)-ethoxy]-benzyl}-thiazolidine-2,4-dione | n.a | n.a | 30 |
| Exemple 5 | 9999 | 9999 | 8 |

Ces résultats montrent l'affinité des composés pour les récepteurs PPAR et plus particulièrement la spécificité de l'affinité des composés de l'invention pour le sous-type PPARγ, comparée à l'affinité des composés pour le sous-type PPARα ou pour le sous-type PPARδ.

### EXEMPLE 13 : COMPOSITIONS

Dans cet exemple, diverses formulations concrètes à base des composés selon l'invention sont illustrées.

### A- VOIE ORALE

| | | |
|---|---|---|
| | (a) Comprimé de 0,2 g | |
| - Composé de l'exemple 5 | | 0,001 g |
| - Amidon | | 0,114 g |
| - Phosphate bicalcique | | 0,020 g |
| - Silice | | 0,020 g |
| - Lactose | | 0,030 g |
| - Talc | | 0,010 g |
| - Stéarate de magnésium | | 0,005 g |
| | (b) Suspension buvable en ampoules de 5 ml | |
| - Composé de l'exemple 4 | | 0,001 g |
| - Glycérine | | 0,500 g |
| - Sorbitol à 70% | | 0,500 g |
| - Saccharinate de sodium | | 0,010 g |
| - Parahydroxybenzoate de méthyle | | 0,040 g |
| - Arome | qs | |
| - Eau purifiée | | qsp5 ml |
| | (c) Comprimé de 0,2 g | |
| - Composé de l'exemple 1 | | 0,050 g |
| - Lactose monohydrate | | 0,132 g |
| - Crospovidone | | 0,007 g |
| - Povidone | | 0,005 g |
| - Aérosil 200 | | 0,004 g |
| - Stéarate de magnésium | | 0,002g |
| | (d) Suspension buvable en ampoules de 10 ml | |
| - Composé de l'exemple 4 | | 0,200 g |
| - Glycérine | | 1,000 g |
| - Sorbitol à 70% | | 1,000 g |
| - Saccharinate de sodium | | 0,010 g |
| - Parahydroxybenzoate de méthyle | | 0,080 g |
| - Arome | | qs |
| - Eau purifiée | qsp | 10 ml |

### B- VOIE TOPIQUE

| | | |
|---|---|---|
| | (a) Onguent | |
| - Composé de l'exemple 5 | | 0,020 g |
| - Myristate d'isopropyle | | 81,700 g |
| - Huile de vaseline fluide | | 9,100 g |
| - Silice ("Aérosil 200") | | 9,180 g |
| | (b) Onguent | |
| - Composé de l'exemple 3 | | 0,300 g |
| - Vaseline blanche codex | qsp | 100 g |
| | (c) Crème Eau-dans-Huile non ionique | |
| - Composé de l'exemple 7 | | 0,100 g |
| - Mélange d'alcools de lanoline émulsifs, de cires | | |
| et d'huiles ("Eucerine anhydre") | | 39,900 g |
| - Parahydroxybenzoate de méthyle | | 0,075 g |
| - Parahydroxybenzoate de propyle | | 0,075 g |
| - Eau déminéralisée stérile | qsp | 100 g |
| | (d) Lotion | |
| - Composé de l'exemple 8 | | 0,100 g |
| - Polyéthylène glycol (PEG 400) | | 69,900 g |
| - Ethanol à 95% | | 30,000 g |
| | (e) Onguent hydrophobe | |
| - Composé de l'exemple 5 | | 0,300 g |
| - Miristate d'isopropyle | | 36,400 g |
| - Huile de silicone ("Rhodorsil 47 V 300") | | 36,400 g |
| - Cire d'abeille | | 13,600 g |
| - Huile de silicone ("Abil 300.000 cst" ) | qsp | 100 g |
| | (f) Crème Huile-dans-Eau non ionique | |
| - Composé de l'exemple 5 | | 1,000 g |
| - Alcool cétylique | | 4,000 g |
| - Monostéarate de glycérole | | 2,500 g |
| - Stéarate de PEG 50 | | 2,500 g |
| - Beurre de karité | | 9,200 g |
| - Propylène glycol | | 2,000 g |
| - Parahydroxybenzoate de méthyle | | 0,075 g |
| - Parahydroxybenzoate de propyle | | 0,075 g |
| - Eau déminéralisée stérile | qsp | 100 g |

## Revendications

1. Composé **caractérisé par le fait qu'**il répond à la formule générale (I) suivante : 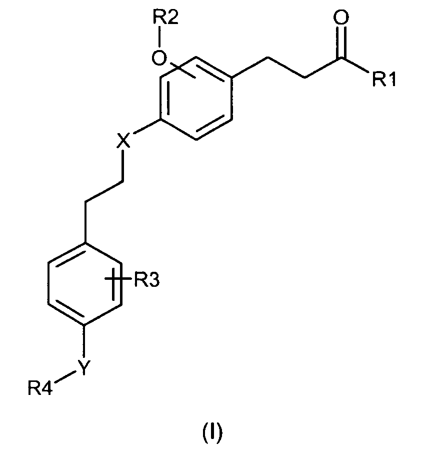 dans laquelle :
- R₁ représente un radical hydroxyle ou un radical alkoxy ;
- R₂ représente un hydrogène, un radical alkyle, un radical cycloalkyle, un radical aralkyle éventuellement substitué ou un radical polyether ;
- R₃ représente un hydrogène, un halogène, un radical alkyle ou un radical alkoxy ;
- R₄ représente un radical alkyle, un radical aryle éventuellement substitué ou un radical aralkyle éventuellement substitué ;
- X représente un atome d'oxygène ou un radical CH₂,
- Y représente un atome d'oxygène, un radical NR₅, un radical OSO₂, OCO, NR₅CO ou NR₅SO₂ ;
- R₅ représente un atome d'hydrogène ou un radical alkyle ;
ainsi que ses sels avec un acide ou une base pharmaceutiquement acceptable, ses solvates pharmaceutiquement acceptables et ses hydrates.

2. Composé selon la revendication 1, **caractérisé par le fait qu'**il se présente sous forme d'un sel d'un métal alcalin ou alcalino-terreux ou d'un sel avec une amine organique.

3. Composé selon la revendication 1, **caractérisé par le fait qu'**il se présente, lorsqu'il possède une fonction amine, sous forme d'un sel d'acide inorganique ou d'un sel d'acide organique.

4. Composé selon l'une quelconque des revendications 1 à 3, **caractérisé par le fait que** ledit radical alkyle représente une chaîne hydrocarbonée saturée, linéaire ou ramifiée, comprenant de 1 à 12 atomes de carbone.

5. Composé selon l'une quelconque des revendications précédentes, **caractérisé par le fait que** ledit radical alkyle sont choisis parmi les radicaux méthyle, éthyle, propyle, isopropyle, butyle, isobutyle, tertiobutyle, pentyle, isoamyle, amyle et hexyle.

6. Composé selon l'une quelconque des revendications précédentes, **caractérisé par le fait que** ledit radical cycloalkyle représente une chaîne hydrocarbonée saturée, cyclique, comprenant de 3 à 7 atomes de carbone.

7. Composé selon l'une quelconque des revendications précédentes, **caractérisé par le fait que** ledit radical aryle éventuellement substitué est choisi parmi un phenyle et un naphtyle éventuellement substitués par un ou plusieurs atomes ou groupes d'atome choisis parmi un alkyle, un alkoxy, un halogène, un hydroxy, un cyano, un trifluorométhyle et un nitro.

8. Composé selon l'une quelconque des revendications précédentes, **caractérisé par le fait que** ledit radical aralkyle éventuellement substitué est choisi parmi les radicaux benzyle et phénéthyle éventuellement substitués par un ou plusieurs atomes ou groupes d'atome choisis parmi un alkyle, un alkoxy, un halogène, un hydroxy, un cyano, un trifluorométhyle et un nitro.

9. Composé selon l'une quelconque des revendications précédentes, **caractérisé par le fait que** ledit atome d'halogène est choisi parmi les atomes de fluor, de chlore, de brome et d'iode.

10. Composé selon l'une quelconque des revendications précédentes, **caractérisé par le fait que** ledit radical alkoxy est choisi parmi les radicaux méthoxy, éthoxy, isopropyloxy, n-propyloxy, tertio-butoxy, n-butoxy, n-pentyloxy et n-hexyloxy.

11. Composé selon l'une quelconque des revendications précédentes, **caractérisé par le fait que** ledit radical polyether est choisi parmi les radicaux ayant de 1 à 7 atomes de carbone interrompus par au moins un atome d'oxygène et de préférence parmi les radicaux méthoxyéthoxy, éthoxyéthoxy, méthoxyéthyle, éthoxyéthyle et méthoxyéthoxyéthoxy.

12. Composé selon la revendication 1, **caractérisé par le fait qu'**il est choisi dans le groupe constitué par :
1. acide 3-{4-[3-(4-benzyloxy-3-methoxy-phenyl)-propyl]-3-butoxy-phenyl}-propanoique
2. acide 3-{3-butoxy-4-[3-(4-ethoxy-3-methoxy-phenyl)-propyl]-phenyl}-propanoique
3. acide 3-{3-butoxy-4-[3-(4-butoxy-3-methoxy-phenyl)-propyl]-phenyl}-propanoique
4. acide 3-{3-butoxy-4-[3-(4-methanesulfonyloxy-3-methoxy-phenyl)-propyl]-phenyl}-propanoique
5. acide 3-(4-{3-[4-(butane-1-sulfonyloxy)-3-methoxy-phenyl]-propyl}-3-butoxy-phenyl)-propanoique
6. acide 3-{3-butoxy-4-[3-(4-ethanesulfonyloxy-3-methoxy-phenyl)-propyl]-phenyl)-propanoique
7. acide 3-[4-{3-[4-(butane-1-sulfonyloxy)-3-methoxy-phenyl]-propyl}-3-(3-fluoro-benzyloxy)-phenyl]-propanoique
8. acide 3-[4-{3-[4-(butane-1-sulfonyloxy)-3-methoxy-phenyl]-propyl} -3-(4-fluoro-benzyloxy)-phenyl]-propanoique
9. acide 3-(4-{3-[4-(butane-1-sulfonyloxy)-3-methoxy-phenyl]-propyl}- 3-cyclopropylmethoxyphenyl)-propanoique
10. acide 3-[4-{3-[4-(butane-1-sulfonyloxy)-3-methoxy-phenyl]-propyl}-2-(3-methoxybenzyloxy)-phenyl]-propanoique
11. acide 3-(4-{3-[4-(butane-1-sulfonyloxy)-3-methoxy-phenyl]-propyl}-2-butoxy-phenyl)-propanoique.
12. 3-[4-[3-(4-acétylamino-phenyl)-propyl]-3-(2-methoxy-ethoxy)-phenyl]-propanoate de méthyle
13. 3-(4-{3-[4-(acétyl-methyl-amino)-phenyl]-propyl}-3-methoxy-phenyl)-propanoate de méthyle
14. acide 3-(4-{3-[4-(butane-1-sulfonyloxy)-phenyl]-propyl}-3-hydroxy-phenyl)-propanoique
15. acide 3-(4-{3-[4-(butane-1-sulfonylamino)-phenyl]-propyl}-3-butoxy-phenyl)-propanoique
16. acide 3-[4-(2-{4-[(3-chloro-benzoyl)-methyl-amino]-phenyl}-ethoxy)-3-(2-ethoxy-ethoxy)-phenyl]-propanoique
17. acide 3-[3-butoxy-4-(2-{4-[methyl-(2-p-tolyl-ethanesulfonyl)-amino]-phenyl}-ethoxy)-phenyl]-propanoique
18. acide 3-(4-{3-[4-(butane-1-sulfonyloxy)-3-methoxy-phenyl]-propyl}-3-butoxy-phenyl)-propanoique
19. 3-{3-butoxy-4-[3-(4-ethoxy-3-fluoro-phenyl)-propyl]-phenyl}-propanoate de méthyle
20. acide 3-[4-{3-[4-(butane-1-sulfonyloxy)-2-methoxy-phenyl]-propyl}-3-(2-ethoxy-ethoxy)-phenyl]-propanoique
21. acide 3-(4-{3-[3-chloro-4-(hexane-1-sulfonyloxy)-phenyl]-propyl}-3-ethoxy-phenyl)-propanoique
22. acide 3-{4-[2-(3-chloro-4-ethoxy-phenyl)-ethoxy]-3-methoxy-phenyl}-propanoique
23. butyrate de 4-{3-[4-(2-carboxy-ethyl)-2-methoxy-phenyl]-propyl}-phenyle

13. Composé selon l'une quelconque des revendications 1 à 12, **caractérisé par le fait qu'**il présente l'une au moins des caractéristiques suivantes :
- R₁ est un radical hydroxyle,
- R₂ représente un radical alkyle ou un radical polyéther,
- R₃ représente un atome d'hydrogène, un radical alkoxy ou un halogène,
- R₄ représente un radical alkyle,
- X représente un atome d'oxygène ou un groupement CH₂,
- Y représente un enchaînement -NR₅SO₂ ou un enchaînement -OSO₂, étant un atome d'hydrogène ou un radical alkyle.

14. Composé selon l'une quelconque des revendications 1 à 12, **caractérisé par le fait qu'**il présente l'une au moins des caractéristiques suivantes :
- R₁ est un radical hydroxyle,
- R₂ représente un radical alkyle inférieur,
- R₃ représente un radical alkoxy inférieur,
- R₄ représente un radical alkyle inférieur,
- X représente un atome d'oxygène ou un groupement CH₂,
- Y représente un enchaînement -OSO₂.

15. Composition cosmétique, **caractérisée par le fait qu'**elle comprend, dans un milieu physiologiquement acceptable, au moins un composé tel que défini à l'une quelconque des revendications 1 à 14.

16. Composition selon la revendication 15, **caractérisée en ce que** la concentration en composé selon l'une des revendications 1 à 14 est comprise entre 0,001 % et 3 % en poids par rapport au poids total de la composition.

17. Utilisation cosmétique d'un composé selon l'une des revendications 1 à 14 ou d'une composition telle que définie à l'une des revendications 15 ou 16 pour l'hygiène corporelle ou capillaire.

18. Composé selon l'une quelconque des revendications 1 à 14 à titre de médicament.

19. Utilisation d'un composé selon l'une quelconque des revendications 1 à 14 dans la fabrication d'un médicament destiné à réguler et/ou à restaurer le métabolisme des lipides cutanés.

20. Utilisation d'un composé selon l'une quelconque des revendications 1 à 14 dans la fabrication d'un médicament destiné au traitement :
- des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle,
- des ichtyoses, des états ichtyosiformes, de la maladie de Darrier, des kératodermies palmoplantaires, des leucoplasies et des états leucoplasiformes, du lichen cutané ou muqueux (buccal),
- des affections dermatologiques avec une composante immuno-allergique inflammatoire, avec ou sans trouble de la prolifération cellulaire, notamment le psoriasis cutané, muqueux ou unguéal, le rhumatisme psoriasique, l'atopie cutanée, telle que l'eczéma, l'atopie respiratoire ou l'hypertrophie gingivale,
- des proliférations dermiques ou épidermiques bénignes ou malignes, d'origine virale ou non, notamment les verrues vulgaires, les verrues planes l'épidermodysplasie verruciforme, les papillomatoses orales ou florides, le lymphome T,
- des proliférations pouvant être induites par les ultra-violets notamment des épithélioma baso et spinocellulaires,
- des lésions précancéreuses cutanées notamment les kératoacanthomes,
- des dermatoses immunes notamment le lupus érythémateux,
- des maladies immunes bulleuses,
- des maladies du collagène notamment la sclérodermie,
- des affections dermatologiques ou générales à composante immunologique,
- de désordres cutanés dus à une exposition aux rayonnements U.V, du vieillissement de la peau, photo-induit ou chronologique ou des pigmentations et des kératoses actiniques, ou toutes pathologies associées au vieillissement chronologique ou actinique notamment la xérose,
- des troubles de la fonction sébacée notamment l'hyperséborrhée de l'acné ou la séborrhée simple,
- des troubles de la cicatrisation ou des vergetures,
- des désordres de la pigmentation, tel l'hyperpigmentation, le mélasma, l'hypopigmentation ou le vitiligo,
- des affections du métabolisme des lipides, tel l'obésité, l'hyperlipidémie, ou le diabète non insulino-dépendant,
- des affections inflammatoires telles que l'arthrite,
- des états cancéreux ou précancéreux,
- de l'alopécie de différentes origines, notamment l'alopécie due à la chimiothérapie ou aux rayonnements,
- des troubles du système immunitaire, tel l'asthme, le diabète sucré de type I, la sclérose en plaque, ou autres disfonctionnements sélectifs du système immunitaire,
- des affections du système cardiovasculaire telles que l'artériosclérose ou l'hypertension.

21. Composition pharmaceutique, **caractérisée par le fait qu'**elle comprend, dans un milieu physiologiquement acceptable, au moins un composé tel que défini à l'une quelconque des revendications 1 à 14.

22. Composition selon la revendication 21, **caractérisée en ce que** la concentration en composé(s) selon l'une des revendications 1 à 14 est comprise entre 0,001 % et 10 % en poids par rapport au poids total de la composition.

23. Composition selon la revendication 21, **caractérisée en ce que** la concentration en composé(s) selon l'une des revendications 1 à 14 est comprise entre 0,01 % et 1 % en poids par rapport au poids total de la composition.

## Claims

1. Compound **characterized in that** it corresponds to the general formula (I) below: 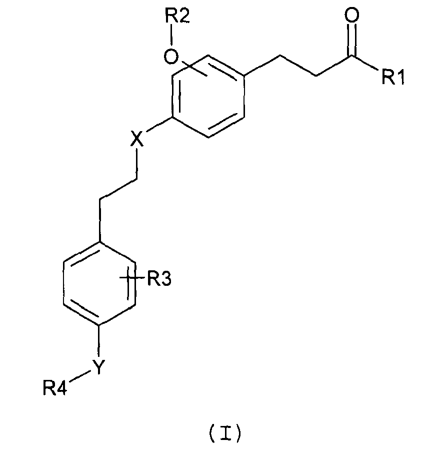 in which:
- **R₁** represents a hydroxyl radical or an alkoxy radical;
- **R₂** represents a hydrogen, an alkyl radical, a cycloalkyl radical, an optionally substituted aralkyl radical or a polyether radical;
- **R₃** represents a hydrogen, a halogen, an alkyl radical or an alkoxy radical;
- **R₄** represents an alkyl radical, an optionally substituted aryl radical or an optionally substituted aralkyl radical;
- **X** represents an oxygen atom or a radical CH₂;
- **Y** represents an oxygen atom, a radical NR₅ or a radical OSO₂, OCO, NR₅CO or NR₅SO₂;
- **R₅** represents a hydrogen atom or an alkyl radical;
and also the salts thereof with a pharmaceutically acceptable acid or base, the pharmaceutically acceptable solvates thereof, and the hydrates thereof.

2. Compound according to Claim 1, **characterized in that** it is in the form of an alkali metal or alkaline-earth metal salt or of a salt with an organic amine.

3. Compound according to Claim 1, **characterized in that**, when it bears an amine function, it is in the form of a mineral acid salt or an organic acid salt.

4. Compound according to any one of Claims 1 to 3, **characterized in that** the said alkyl radical represents a linear or branched saturated hydrocarbon-based chain containing from 1 to 12 carbon atoms.

5. Compound according to any one of the preceding claims, **characterized in that** the said alkyl radical is chosen from methyl, ethyl, propyl, isopropyl, butyl, isobutyl, tert-butyl, pentyl, isoamyl, amyl and hexyl radicals.

6. Compound according to any one of the preceding claims, **characterized in that** the said cycloalkyl radical represents a saturated cyclic hydrocarbon-based chain containing from 3 to 7 carbon atoms.

7. Compound according to any one of the preceding claims, **characterized in that** the said optionally substituted aryl radical is chosen from phenyl and naphthyl optionally substituted with one or more atoms or groups of atoms chosen from an alkyl, an alkoxy, a halogen, a hydroxyl, a cyano, a trifluoromethyl and a nitro.

8. Compound according to any one of the preceding claims, **characterized in that** the said optionally substituted aralkyl radical is chosen from benzyl and phenethyl optionally substituted with one or more atoms or groups of atoms chosen from an alkyl, an alkoxy, a halogen, a hydroxyl, a cyano, a trifluoromethyl and a nitro.

9. Compound according to any one of the preceding claims, **characterized in that** the said halogen atom is chosen from fluorine, chlorine, bromine and iodine atoms.

10. Compound according to any one of the preceding claims, **characterized in that** the said alkoxy radical is chosen from methoxy, ethoxy, isopropyloxy, n-propyloxy, tert-butoxy, n-butoxy, n-pentyloxy and n-hexyloxy radicals.

11. Compound according to any one of the preceding claims, **characterized in that** the said polyether radical is chosen from radicals containing from 1 to 7 carbon atoms interrupted with at least one oxygen atom, and preferably methoxyethoxy, ethoxyethoxy, methoxyethyl, ethoxyethyl and methoxyethoxyethoxy radicals.

12. Compound according to Claim 1, **characterized in that** it is chosen from the group formed by:
1. 3-{4-[3-(4-benzyloxy-3-methoxyphenyl)propyl]-3-butoxyphenyl}propanoic acid
2. 3-{3-butoxy-4-[3-(4-ethoxy-3-methoxyphenyl)-propyl]phenyl}propanoic acid
3. 3-{3-butoxy-4-[3-(4-butoxy-3-methoxyphenyl)-propyl]phenyl}propanoic acid
4. 3-{3-butoxy-4-[3-(4-methanesulfonyloxy-3-methoxyphenyl)propyl]phenyl}propanoic acid
5. 3-(4-{3-[4-(butane-1-sulfonyloxy)-3-methoxyphenyl]propyl}-3-butoxyphenyl)propanoic acid
6. 3-{3-butoxy-4-[3-(4-ethanesulfonyloxy-3-methoxyphenyl)propyl]phenyl)propanoic acid
7. 3-[4-{3-[4-(butane-1-sulfonyloxy)-3-methoxyphenyl]propyl}-3-(3-fluorobenzyloxy)phenyl]propanoic acid
8. 3-[4-{3-[4-(butane-1-sulfonyloxy)-3-methoxyphenyl]propyl}-3-(4-fluorobenzyloxy)phenyl]propanoic acid
9. 3-(4-{3-[4-(butane-1-sulfonyloxy)-3-methoxyphenyl]propyl}-3-cyclopropylmethoxyphenyl)propanoic acid
10. 3-[4-{3-[4-(butane-1-sulfonyloxy)-3-methoxyphenyl]propyl}-2-(3-methoxybenzyloxy)phenyl]propanoic acid
11. 3-(4-{3-[4-(butane-1-sulfonyloxy)-3-methoxyphenyl]propyl}-2-butoxyphenyl)propanoic acid.
12. methyl 3-[4-[3-(4-acetylaminophenyl)propyl]-3-(2-methoxyethoxy)phenyl]propanoate
13. methyl 3-(4-{3-[4-(acetylmethylamino)phenyl]-propyl}-3-methoxyphenyl)propanoate
14. 3-(4-{3-[4-(butane-1-sulfonyloxy)phenyl]-propyl}-3-hydroxyphenyl)propanoic acid
15. 3-(4-{3-[4-(butane-1-sulfonylamino)phenyl]-propyl}-3-butoxyphenyl)propanoic acid
16. 3-[4-(2-{4-[(3-chlorobenzoyl)methylamino]-phenyl}ethoxy)-3-(2-ethoxyethoxy)phenyl]propanoic acid
17. 3-[3-butoxy-4-(2-{4-[methyl-(2-p-tolylethanesulfonyl)amino]phenyl}ethoxy)phenyl]propanoic acid
18. 3-(4-{3-[4-(butane-1-sulfonyloxy)-3-methoxyphenyl]propyl}-3-butoxyphenyl)propanoic acid
19. methyl 3-{3-butoxy-4-[3-(4-ethoxy-3-fluorophenyl)propyl]phenyl}propanoate
20. 3-[4-{3-[4-(butane-1-sulfonyloxy)-2-methoxyphenyl]propyl}-3-(2-ethoxyethoxy)phenyl]propanoic acid
21. 3-(4-{3-[3-chloro-4-(hexane-1-sulfonyloxy)-phenyl]propyl}-3-ethoxyphenyl)propanoic acid
22. 3-{4-[2-(3-chloro-4-ethoxyphenyl)ethoxy]-3-methoxyphenyl}propanoic acid
23. 4-{3-[4-(2-carboxyethyl)-2-methoxyphenyl]-propyl}phenyl butyrate.

13. Compound according to any one of Claims 1 to 12, **characterized in that** it has at least one of the following characteristics:
- R₁ is a hydroxyl radical,
- R₂ represents an alkyl radical or a radical polyether,
- R₃ represents a hydrogen atom, an alkoxy radical or a halogen,
- R₄ represents an alkyl radical,
- X represents an oxygen atom or a group CH₂,
- Y represents a sequence -NR₅SO₂ or a sequence -OSO₂, R₅ being a hydrogen atom or an alkyl radical.

14. Compound according to any one of Claims 1 to 12, **characterized in that** it has at least one of the following characteristics:
- R₁ is a hydroxyl radical,
- R₂ represents a lower alkyl radical,
- R₃ represents a lower alkoxy radical,
- R₄ represents a lower alkyl radical,
- X represents an oxygen atom or a group CH₂,
- Y represents a sequence -OSO₂.

15. Cosmetic composition, **characterized in that** it comprises, in a physiologically acceptable support, at least one of the compounds as defined in any one of Claims 1 to 14.

16. Composition according to Claim 15, **characterized in that** the concentration of compound(s) according to one of Claims 1 to 14 is between 0.001% and 3% by weight relative to the total weight of the composition.

17. Cosmetic use of a composition as defined in one of Claims 1 to 14 or of a composition as defined in either of Claims 15 and 16, for body or hair hygiene.

18. Compound according to any one of Claims 1 to 14 as a medicament.

19. Use of a compound according to any one of Claims 1 to 14 in the manufacture of a composition for regulating and/or restoring skin lipid metabolism.

20. Use of a compound according to any one of Claims 1 to 14 in the manufacture of a composition for treating:
- dermatological complaints associated with a keratinization disorder relating to cell differentiation and proliferation, especially for treating common acne, comedones, polymorphs, acne rosacea, nodulocystic acne, acne conglobata, senile acne, and secondary acnes such as solar acne, medication-related acne or occupational acne;
- ichthyosis, ichthyosiform conditions, Darier's disease, palmoplantar keratoderma, leukoplakia and leukoplakiform conditions, and cutaneous or mucous (buccal) lichen;
- dermatological complaints with an inflammatory immunoallergic component, with or without cell proliferation disorder, especially cutaneous, mucous or ungual psoriasis, psoriatic rheumatism, cutaneous atopy, such as eczema, respiratory atopy, or gingival hypertrophy;
- dermal or epidermal proliferations, whether benign or malignant, and whether of viral origin or otherwise, especially common warts, flat warts and verruciform epidermodysplasia, oral or florid papillomatoses, T lymphoma;
- proliferations that may be induced by ultraviolet radiation, especially basal cell and spinal cell epithelioma;
- precancerous skin lesions, especially keratoacanthomas;
- immune dermatoses, especially lupus erythematosus;
- immune bullous diseases;
- collagen diseases, especially scleroderma;
- dermatological or general complaints with an immunological component;
- skin disorders caused by exposure to UV radiation, photoinduced or chronological ageing of the skin, actinic pigmentations and keratosis, or any pathology associated with chronological or actinic ageing, especially xerosis;
- sebaceous function disorders, especially the hyperseborrhoea of acne or simple seborrhoea;
- cicatrization disorders or stretchmarks;
- pigmentation disorders, such as hyperpigmentation, melasma, hypopigmentation or vitiligo;
- lipid metabolism complaints, such as obesity, hyperlipidaemia, or non-insulin-dependent diabetes;
- inflammatory complaints such as arthritis;
- cancerous or precancerous conditions;
- alopecia of various origins, especially alopecia caused by chemotherapy or radiation;
- disorders of the immune system, such as asthma, type I sugar diabetes, multiple sclerosis or other selective dysfunctions of the immune system; and
- complaints of the cardiovascular system, such as arteriosclerosis or hypertension.

21. Pharmaceutical composition, **characterized in that** it comprises, in a physiologically acceptable medium, at least one compound as defined in any one of Claims 1 to 14.

22. Composition according to Claim 21, **characterized in that** the concentration of compound(s) according to one of Claims 1 to 14 is between 0.001% and 10% by weight relative to the total weight of the composition.

23. Composition according to Claim 21, **characterized in that** the concentration of compound(s) according to one of Claims 1 to 14 is between 0.01% and 1% by weight relative to the total weight of the composition.

## Patentansprüche

1. Verbindung, **dadurch gekennzeichnet, dass** sie der folgenden allgemeinen Formel entspricht: 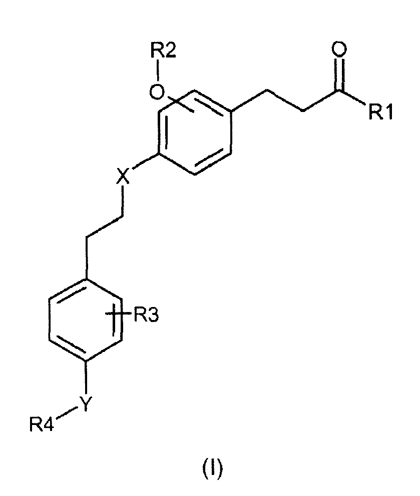 in der Formel:
• R₁ bedeutet eine Hydroxygruppe oder eine Alkoxygruppe;
• R₂ bedeutet ein Wasserstoffatom, eine Alkylgruppe, eine Cycloalkylgruppe, eine Aralkylgruppe, die gegebenenfalls substituiert ist, oder eine Polyethergruppe;
• R₃ bedeutet ein Wasserstoffatom, ein Halogenatom, eine Alkylgruppe oder eine Alkoxygruppe;
• R₄ bedeutet eine Alkylgruppe, eine Arylgruppe, die gegebenenfalls substituiert ist, oder eine Aralkylgruppe, die gegebenenfalls substituiert ist;
• X bedeutet ein Sauerstoffatom oder eine Gruppe CH₂;
• Y bedeutet ein Sauerstoffatom, eine Gruppe NR₅, eine Gruppe OSO₂, OCO, NR₅CO oder NR₅SO₂;
• R₅ bedeutet ein Wasserstoffatom oder eine Alkylgruppe;
sowie ihre Salze mit einer pharmazeutisch akzeptablen Säure oder Base, ihre pharmazeutisch akzeptablen Solvate und ihre Hydrate.

2. Verbindung nach Anspruch 1, **dadurch gekennzeichnet, dass** sie in Form eines Salzes eines Alkalimetalls oder Erdalkalimetalls oder eines Salzes mit einem organischen Amin vorliegt.

3. Verbindung nach Anspruch 1, **dadurch gekennzeichnet, dass** sie in der Form eines Salzes einer anorganischen Säure oder eines Salzes einer organischen Säure vorliegt, wenn sie eine Aminofunktion aufweist.

4. Verbindung nach einem der Ansprüche 1 bis 3, **dadurch gekennzeichnet, dass** die Alkylgruppe eine gesättigte, lineare oder verzweigte Kohlenwasserstoffgruppe mit 1 bis 12 Kohlenstoffatomen bedeutet.

5. Verbindung nach einem der vorhergehenden Ansprüche, **dadurch gekennzeichnet, dass** die Alkylgruppe unter den Gruppen Methyl, Ethyl, Propyl, Isopropyl, Butyl, Isobutyl, *tert-*Butyl, Pentyl, Isoamyl, Amyl und Hexyl ausgewählt ist.

6. Verbindung nach einem der vorhergehenden Ansprüche, **dadurch gekennzeichnet, dass** die Cycloalkylgruppe eine gesättigte, cyclische Kohlenwasserstoffgruppe mit 3 bis 7 Kohlenstoffatomen bedeutet.

7. Verbindung nach einem der vorhergehenden Ansprüche, **dadurch gekennzeichnet, dass** die gegebenenfalls substituierte Arylgruppe unter einer Phenylgruppe und einer Naphthylgruppe ausgewählt ist, die gegebenenfalls mit einem oder mehreren Atomen oder einer oder mehreren Atomgruppierungen substituiert sind, die unter Alkyl, Alkoxy, Halogen, Hydroxy, Cyano, Trifluormethyl und Nitro ausgewählt sind.

8. Verbindung nach einem der vorhergehenden Ansprüche, **dadurch gekennzeichnet, dass** die gegebenenfalls substituierte Aralkylgruppe unter einer Benzylgruppe und einer Phenethylgruppe ausgewählt ist, die gegebenenfalls mit einem oder mehreren Atomen oder einer oder mehreren Atomgruppierungen substituiert sind, die unter Alkyl, Alkoxy, Halogen, Hydroxy, Cyano, Trifluormethyl und Nitro ausgewählt sind.

9. Verbindung nach einem der vorhergehenden Ansprüche, **dadurch gekennzeichnet, dass** das Halogenatom unter Fluor, Chlor, Brom und Iod ausgewählt ist.

10. Verbindung nach einem der vorhergehenden Ansprüche, **dadurch gekennzeichnet, dass** die Alkoxygruppe unter den Gruppen Methoxy, Ethoxy, Isopropyloxy, n-Propyloxy, *tert-*Butoxy, n-Butoxy, n-Pentyloxy und Hexyloxy ausgewählt ist.

11. Verbindung nach einem der vorhergehenden Ansprüche, **dadurch gekennzeichnet, dass** der Polyether unter den Gruppen mit 1 bis 7 Kohlenstoffatomen, die durch mindestens ein Sauerstoffatom unterbrochen sind, und vorzugsweise unter den Gruppen Methoxyethoxy, Ethoxyethoxy, Methoxyethyl, Ethoxyethyl, und Methoxyethoxyethoxy ausgewählt ist.

12. Verbindung nach Anspruch 1, **dadurch gekennzeichnet, dass** sie unter den folgenden Verbindungen ausgewählt ist:
1. 3-{4-[3-(4-Benzyloxy-3-methoxy-phenyl)-propyl]-3-butoxyphenyl}-propansäure;
2. 3-{3-Butoxy-4-[3-(4-ethoxy-3-methoxy-phenyl)-propyl]-phenyl}-propansäure;
3. 3-{3-Butoxy-4-[3-(4-butoxy-3-methoxy-phenyl)-propyl]-phenyl}-propansäure;
4. 3-{3-Butoxy-4-[3-(4-methanesulfonyloxy-3-methoxy-phenyl)-propyl]-phenyl}-propansäure;
5. 3-(4-(3-[4-(Butan-1-sulfonyloxy)-3-methoxy-phenyl]-propyl)-3-butoxy-phenyl)-propansäure;
6. 3-{3-Butoxy-4-[3-(4-ethanesulfonyloxy-3-methoxy-phenyl)-propyl]-phenyl)-propansäure;
7. 3-[4-{3-[4-(Butan-1-sulfonyloxy)-3-methoxy-phenyl]-propyl}-3-(3-fluor-benzyloxy)-phenyl]-propansäure;
8. 3-[4-{3-[4-(Butan-1-sulfonyloxy)-3-methoxy-phenyl]-propyl}-3-(4-fluorbenzyloxy)-phenyl]-propansäure;
9. 3-(4-{3-[4-(Butan-1-sulfonyloxy)-3-methoxy-phenyl]-propyl}-3-cyclopropylmethoxyphenyl)-propansäure;
10. 3-[4-{3-[4-(Butan-1-sulfonyloxy)-3-methoxy-phenyl]-propyl}-2-(3-methoxybenzyloxy)-phenyl]-propansäure;
11. 3-(4-{3-[4-(Butan-1-sulfonyloxy)-3-methoxy-phenyl]-propyl}-2-butoxy-phenyl)-propansäure;
12. Methyl-3-[4-[3-(4-acetylamino-phenyl)-propyl]-3-(2-methoxyethoxy)-phenyl]-propanoat
13. Methyl-3-(4-{3-[4-(acetyl-methyl-amino)-phenyl]-propyl}-3-methoxy-phenyl)-propanoat
14. 3-(4-{3-[4-(Butan-1-sulfonyloxy)-phenyl]-propyl}-3-hydroxyphenyl)-propansäure;
15. 3-(4-{3-[4-(Butan-1-sulfonylamino)-phenyl]-propyl}-3-butoxyphenyl)-propansäure;
16. 3-[4-(2-{4-[(3-Chlor-benzoyl)-methyl-amino]-phenyl}-ethoxy)-3-(2-ethoxy-ethoxy)-phenyl]-propansäure;
17. 3-[3-Butoxy-4-(2-{4-[methyl-(2-p-tolyl-ethansulfonyl)-amino]-phenyl}-ethoxy)-phenyl]-propansäure;
18. 3-(4-{3-[4-(Butan-1-sulfonyloxy)-3-methoxy-phenyl]-propyl}-3-butoxy-phenyl)-propansäure;
19. Methyl-3-{3-butoxy-4-[3-(4-ethoxy-3-fluor-phenyl)-propyl]-phenyl}-propanoat
20. 3-[4-{3-[4-(Butan-1-sulfonyloxy)-2-methoxy-phenyl]-propyl}-3-(2-ethoxy-ethoxy)-phenyl]-propansäure;
21. 3-(4-{3-[3-Chlor-4-(hexan-1-sulfonyloxy)-phenyl]-propyl}-3-ethoxy-phenyl)-propansäure;
22. 3-{4-[2-(3-Chlor-4-ethoxy-phenyl)-ethoxy]-3-methoxyphenyl}-propansäure;
23. 4-{3-[4-(2-Carboxy-ethyl)-2-methoxy-phenyl]-propyl}-phenyl-butyrat.

13. Verbindung nach einem der Ansprüche 1 bis 12, **dadurch gekennzeichnet, dass** sie mindestens eines der folgenden Merkmale aufweist:
• R₁ bedeutet eine Hydroxygruppe;
• R₂ bedeutet eine Alkylgruppe oder eine Polyethergruppe;
• R₃ bedeutet ein Wasserstoffatom, eine Alkoxygruppe oder ein Halogenatom;
• R₄ bedeutet eine Alkylgruppe;
• X bedeutet ein Sauerstoffatom oder eine Gruppe CH₂;
• Y bedeutet eine Verknüpfung -NR₅SO₂ oder eine Verknüpfung -OSO₂, wobei ein Wasserstoffatom oder eine Alkylgruppe ist.

14. Verbindung nach einem der Ansprüche 1 bis 12, **dadurch gekennzeichnet, dass** sie mindestens eines der folgenden Merkmale aufweist:
• R₁ bedeutet eine Hydroxygruppe;
• R₂ bedeutet eine niedere Alkylgruppe;
• R₃ bedeutet eine niedere Alkoxygruppe;
• R₄ bedeutet eine niedere Alkylgruppe;
• X bedeutet ein Sauerstoffatom oder eine Gruppe CH₂;
• Y bedeutet eine Verknüpfung -OSO₂.

15. Kosmetische Zusammensetzung, **dadurch gekennzeichnet, dass** sie in einem physiologisch akzeptablen Medium mindestens eine Verbindung enthält, wie sie in einem der Ansprüche 1 bis 14 definiert ist.

16. Zusammensetzung nach Anspruch 15, **dadurch gekennzeichnet, dass** die Konzentration der Verbindung nach einem der Ansprüche 1 bis 14 im Bereich von 0,001 bis 3 Gew.-%, bezogen auf das Gesamtgewicht der Zusammensetzung, liegt.

17. Kosmetische Verwendung einer Verbindung nach einem der Ansprüche 1 bis 14 oder einer Zusammensetzung, wie sie in einem der Ansprüche 15 oder 16 definiert ist, für die Körper- oder Haarpflege.

18. Verbindung nach einem der Ansprüche 1 bis 14 als Arzneimittel.

19. Verwendung einer Verbindung nach einem der Ansprüche 1 bis 14 für die Herstellung eines Arzneimittels, das dazu vorgesehen ist, den Metabolismus der Hautlipide zu regulieren und/oder wiederherzustellen.

20. Verwendung einer Verbindung nach einem der Ansprüche 1 bis 14 für die Herstellung eines Arzneimittels, das vorgesehen ist:
• zur Behandlung von dermatologischen Erkrankungen, die mit einer Keratinisierungsstörung zusammenhängen, die auf der Differenzierung und Proliferation beruht, insbesondere zur Behandlung von Acne vulgaris, Acne comedonica, polymorpher Akne, Acne rosacea, nodulocystischer Akne, Acne conglobata, Acne senilis und sekundären Akneformen, wie Acne solaris, Acne medicamentosa oder Acne professionalis;
• Ichthyosis, ichtyosisartigen Zuständen, der Darier Krankheit, Palmoplantarkeratosen, Leukoplakien und leucoplakieformen Zuständen oder Lichen der Haut oder der Schleimhäute (buccal);
• zur Behandlung dermatologischer Erkrankungen mit immunoallergischer entzündlicher Komponente mit oder ohne Störung der Zellproliferation, insbesondere von Psoriasis der Haut, der Schleimhäute oder der Nägel, Psoriasis arthropathica, Atopie der Haut, wie Ekzemen, Atopie der Atemwege oder Hypertrophie des Zahnfleisches;
• zur Behandlung von Proliferationen der Dermis oder Epidermis, die gutartig oder bösartig und gegebenenfalls viralen Ursprungs sein können, wie Verrucae vulgares, Verrucae planae und Epidermodysplasia verruciformis, Papillomatosis oralis oder florida, T-Lymphom;
• zur Behandlung von Proliferationen, die durch UV-Strahlung hervorgerufen werden, insbesondere Epithelioma basocellulare und spinocellulare;
• zur Behandlung von präkanzerösen Hautläsionen, insbesondere Keratoacanthomen;
• zur Behandlung von Immundermatosen, insbesondere Lupus erythematodes;
• zur Behandlung von bullösen Immunerkrankungen;
• zur Behandlung von Kollagenerkrankungen wie Sklerodermie;
• zur Behandlung von dermatologischen oder allgemeinen Erkrankungen mit immunologischer Komponente;
• zur Behandlung von Hautstörungen, die mit einer UV-Exposition zusammenhängen, der Hautalterung, die lichtinduziert oder altersbedingt sein kann, Pimentierungen und aktinischen Keratosen oder beliebigen Erkrankungen, die mit einer altersbedingten oder aktinischen Alterung zusammenhängen, insbesondere Xerose;
• zur Behandlung von Funktionsstörungen der Talgdrüsen, wie Hyperseborrhoe bei Akne oder Seborrhoe simplex;
• zur Behandlung von Störungen der Wundheilung oder von Streifen;
• zur Behandlung von Pigmentierungsstörungen, wie Hyperpigmentierung, Melasma, Hypopigmentierung oder Vitiligo;
• zur Behandlung von Erkrankungen des Lipidmetabolismus, wie Adipositas, Hyperlipidämie oder nicht-insulinpflichtigem Diabetes;
• zur Behandlung von entzündlichen Erkrankungen, wie Arthritis;
• zur Behandlung von kanzerösen oder präkanzerösen Zuständen;
• zur Behandlung von Alopezie unterschiedlicher Ursache, insbesondere durch Chemotherapie oder Strahlung verursachter Alopezie;
• zur Behandlung von Störungen des Immunsystems, wie Asthma, Diabetes Typ I, multipler Sklerose oder weiteren selektiven Funktionsstörungen des Immunsystems;
• zur Behandlung von Erkrankungen des cardiovaskulären Systems, wie Arteriosklerose oder Bluthochdruck.

21. Pharmazeutische Zusammensetzung **dadurch gekennzeichnet, dass** sie in einem physiologisch akzeptablen Medium mindestens eine Verbindung enthält, wie sie in einem der Ansprüche 1 bis 14 definiert ist.

22. Zusammensetzung nach Anspruch 21, **dadurch gekennzeichnet, dass** die Konzentration der Verbindung(en) nach einem der Ansprüche 1 bis 14 im Bereich von 0,001 bis 10 Gew.-%, bezogen auf das Gesamtgewicht der Zusammensetzung, liegt.

23. Zusammensetzung nach Anspruch 21, **dadurch gekennzeichnet, dass** die Konzentration der Verbindung(en) nach einem der Ansprüche 1 bis 14 im Bereich von 0,01 bis 1 Gew.-%, bezogen auf das Gesamtgewicht der Zusammensetzung, liegt.
